# EUROPEAN PATENT APPLICATION

(11) **EP 2 939 687 A1**
(43) Date of publication of application: **04.11.2015**
(21) Application number: 15167603.8
(22) Date of filing: 07.11.2007
(51) Int. Cl.: A61K 38/47, A61K 38/44, A61K 31/573, A61K 31/58

(54) **USE OF HYDROLYTIC ENZYMES TO DISSOLVE BIOFILM IN EARS**

(30) Priority: 01.12.2006 US 868131 P; 15.12.2006 US 870328 P
(62) Divisional of application: 07864012.5
(71) Applicant: Laclede, Inc., Rancho Dominguez, CA 90220 (US)
(72) Inventor: Pellico, Michael, Rancho Dominguez, CA California 90220 (US)
(74) Representative: Winter, Brandl, Fürniss, Hübner, Röss, Kaiser, Polte - Partnerschaft mbB

(57) **Abstract**

A composition for removal of biofilm in the ears is useful for the treatment of ear infections such as otitis media, particularly those infections caused by *Pseudomonas aeruginosa.* In general, the composition comprises: (1) a quantity of at least one enzyme that catalyzes the hydrolysis of a bond that connects two monosaccharides in a polysaccharide or that connects a monosaccharide with a protein molecule in a glycoprotein sufficient to break down biofilm in the ear; and (2) a pharmaceutically acceptable carrier suitable for administration into the ear canal. The composition can further include ingredients such as a steroid, lysozyme, lactoferrin, or a peroxidase; if a peroxidase is included, the composition can further include an oxidase to generate peroxide as well as a substrate for the oxidase. The composition can be used in methods for treatment of an ear infection based on the ability of the composition to dissolve biofilm in the ear.

## Description

### CROSS-REFERENCES

This application claims priority from Provisional Application Serial No. 60/868,131 by Michael Pellico, entitled "Use of Hydrolytic and Oxidative Enzymes to Dissolve Biofilm in Ears," filed December 1, 2006, and from Provisional Application Serial No. 60/870,328 by Michael Pellico, entitled "Use of Hydrolytic and Oxidative Enzymes to Dissolve Biofilm in Ears," filed December 15, 2006, both of which are incorporated herein in their entirety by this reference.

### FIELD OF THE INVENTION

This invention relates to ear cleaning compositions where the cleaning composition contains biofilm dissolving enzymes, in particular hydrolytic enzymes and oxidative enzymes.

### BACKGROUND OF THE INVENTION

Ear cleaning compositions are formulated to contain ingredients for the prevention and treatment of ear infections.

Otitis media and otitis externa are particularly common ear infections. Although common, these ear infections, including otitis media and otitis externa, can have severe consequences. Three out of four children experience otitis media by the time that they are three years old.

There are two main types of otitis media. The first type is called acute otitis media (AOM). Parts of the ear are infected and swollen. Fluid and mucus are trapped inside the ear. AOM can be very painful.

The second type is called otitis media with effusion(fluid) or OME. This means that fluid and mucus stay trapped in the ear after the infection is thought to be over. OME often leads to new infections and can affect a child's hearing. Hearing loss, especially in children, can impair learning capacity and even delay speech development. Otitis media is also serious because the infection can spread to nearby structures in the head, especially the mastoid. Infections of the mastoid are often extremely difficult to treat, even with broad-spectrum antibiotics.

Otitis media or otitis externa usually happens when viruses, bacteria, or yeast/fungi find their way into the middle or external ear canal and cause an infection. Often after the acute infection has passed, the effusion remains and becomes chronic, lasting for weeks, months, or even years. This condition makes one subject to frequent recurrences of the acute infection.

The viruses that cause ear infections are thought to be the same ones that cause upper respiratory infections such as influenza or coryza (the common cold). One of the bacteria associated with ear infection is *Pseudomonas aeruginosa.* It is resistant to almost every possible antibiotic. The unfortunate tendency is for most bacteria to be killed off, leaving infection with the very resistant and practically immortal *P. aeruginosa.*

Current treatment for antibiotics is still with antibiotics, antiseptics, and fungicides. More often now, however, doctors are taking a wait and see attitude. Part of the reason that antibiotics have come into disfavor for treating ear infections is the fear that overuse of antibiotics will lead to a proliferation of antibiotic-resistant bacteria. It has been known for many years that bacteria have the ability to spread antibiotic resistance from one species to another through the action of plasmids known as resistance transfer factors (RTFs). Thus, if a relatively harmless commensal such as *Escherichia coli* becomes resistant to a particular antibiotic administered to a patient in response to an ear infection, the resistance to that antibiotic can be spread to other bacterial species such as the much more pathogenic P. *aeruginosa,* and once spread can be inherited in P. *aeruginosa.* Of particular significance is the generation of plasmids that carry multiple resistance genes. These mechanisms are described in A.A. Sayers and D.D. Whitt, "Bacterial Pathogenesis: A Molecular Approach" (ASM Press, Washington, DC 1994), pp. 107-109, incorporated herein by this reference.

What has been shown repeatedly is that the resistance rate of common bacteria in countries that universally prescribe antibiotics for ear infections is much higher than in countries that do not routinely prescribe antibiotics. For example, researchers have found that 56% of children who had been prescribed antibiotics harbored multidrug-resistant bacteria in their noses, compared with 28% of the others.

The reason that antibiotics are frequently ineffective in this clinical situation is that recently it has been discovered that bacteria are living in a dormant state inside a slimy biofilm. The seemingly innocuous fluid behind the ear is actually a microbe-laden biofilm containing bacteria that become activated and grow rapidly under the right circumstances. This biofilm is also in the outer ear canal causing recurrent chronic otitis externa, resistant to most antibiotics. Otitis externa is typically associated with discomfort that is limited to the external auditory canal, with erythema (redness), and with swelling of the canal with variable discharge. Excessive moisture and trauma can predispose an individual to the occurrence of otitis externa. Otitis externa can be disabling enough to cause a significant fraction of patients to interrupt their daily activities for several days, typically requiring bed rest. If otitis externa is not optimally treated, especially in immunocompromised patients, the potentially life-threatening infection can spread to the surrounding tissues with extremely serious consequences. Immunocompromised patients include those patients with an immune system deficiency such as that occurring as a result of HIV infection, and those patients taking immunosuppressants such as tacrolimus to prevent transplant rejection. Otitis externa can occur in conjunction with otitis media; the latter can result from the spread of otitis externa. Like otitis media, otitis externa can spread into the mastoid and generate an extremely serious infection, one that can have a mortality rate exceeding 50%.

This revised understanding has come about because, previously, scientists studied bacteria in their free-floating form. Bacteria prefer the slimy, communal life because it protects them from toxins in the environment. Biofilm formation takes place in a step by step manner. First, inorganic or organic molecules are adsorbed to a surface. This creates a conditioning layer that increases the ability of bacteria to attach to a surface. Once a conditioning layer is formed, bacterial adhesion follows. Live or dead cells will attach to surfaces with similar propensity. Bacterial attachment is mediated by fimbriae, pili, and flagella, and by extracellular polysaccharides.

When first formed, the bond between the conditioning layer and the bacteria is not strong and can be easily removed. With time, however, these bonds are strengthened making removal difficult. Once embedded within a biofilm, bacterial cells have an opportunity to repair cellular damage and to metabolize nutrients within the biofilm. As the biofilm continues to grow, the extracellular polysaccharides provide more and more protection. A biofilm is mature within 24 hours. Biofilm development can occur within one hour. After an eight-hour period, more than 91% of the bacteria are strongly attached within the biofilm. Killing bacteria within a biofilm requires up to 1000 times more antibiotic than is required to kill free-floating bacteria. The film physically prevents the antibiotic from reaching the bacteria. In addition, most bacteria in the biofilm are dormant and antibiotics typically only kill bacteria that are actively dividing.

The eardrum is coated with a slimy reservoir of hibernating bacteria. These inactive bacteria do not cause symptoms of an active infection but eventually they slough off and become free-floating active bacteria and cause another infection. This is one of the significant factors behind the existence of recurrent infections in such patients. Data show that bacteria incorporated in biofilms are more resistant that single cells and this is believed to be caused by physical protection by the biofilm matrix or by altered physiology of bacterial cells in the biofilm.

Bacteria have a natural tendency to attach to surfaces and to initiate the formation of a biofilm. The biofilm matrix is a collection of microcolonies with water channels in between and an assortment of cells and extracellular polymers such as polysaccharides, glycoproteins, and proteins. The different types of bonds between the saccharides give rise to a large number of different classes of polysaccharides including levans, dextrans, cellulose, glycogen, and alginates. Bacteria have the capacity to attach to and to colonize the surface of most materials. Attachment often results in the production of extracellular polysaccharides and changes in cellular morphology and growth rates. Different genes are expressed in bacteria that are attached to surfaces as compared to planktonic bacteria. As a result, surface-attached bacteria display increased resistance to toxic chemicals and biocides. While biocides have proven effective in killing free-floating bacteria, they are not effective in destroying bacteria within a biofilm. It becomes imperative that the biofilm be destroyed before the biocides can become effective.

There are many methods known to remove biofilms. The methods that are used to remove biofilm include the use of hypochlorite, hydrogen peroxide, ozone, detergents, or acids, the application of heat, the use of mechanical activity, or the use of ultrasound. Combinations of these methods are also used.

Many of these methods, although effective, are not suitable for use on biofilms that form on the body or within the body. These methods are too harsh and disruptive of tissue for use in this context. A safe method is required to remove biofilms that form on the body or within the body.

Enzymes have been used to dissolve biofilms before, but not in the context of biofilms that form on the body or within the body. In laundry detergents, enzymes are used to remove deposits that may, in fact, be biofilms. Contact lens solutions use enzymes to remove the biofilm that can grow on a contact lens. In the dental field, dextranase and mutanase are used to remove plaque, a biofilm, from teeth.

Accordingly, there is a need for an improved method for removing biofilms that form on the body or within the body, particularly behind the eardrum. The improved method should be effective and safe. The improved method should also be compatible with antibiotics and other treatments for bacterial infection.

### SUMMARY OF THE INVENTION

This invention is directed to compositions that have the activity of removing biofilm, particularly in the ear. Compositions and methods according to the present invention are suitable for treatment of ear infections such as otitis externa and otitis media, particularly those caused by *Pseudomonas aeruginosa.*

One aspect of the present invention is a composition for removal of biofilm in the ear comprising:
(1) a quantity of at least one enzyme that catalyzes the hydrolysis of a bond that connects two monosaccharides in a polysaccharide or that connects a monosaccharide with a protein molecule in a glycoprotein sufficient to break down biofilm in the ear; and
(2) a pharmaceutically acceptable carrier suitable for administration into the ear canal.

The at least one enzyme that catalyzes the hydrolysis of a bond that connects two monosaccharides in a polysaccharide or that connects a monosaccharide with a protein molecule in a glycoprotein can be selected from the group consisting of xylanase, β-glucanase, cellulase, α-galactosidase, glucanases, amylase, hyaluronidase, polygalacturonase (pectinase), dextranase, cellobiohydrolase, pullulanase, glycosylceramidase, glucan 1,4-α-glucosidase, oligo-1,6-glucosidase, fucoidanase, glycosylceramidase, glycosylceramidase, thioglucosidase, and glycopeptide N-glycosidase. Typically, the enzyme is selected from the group consisting of xylanase, β-glucanase, cellulase, α-galactosidase, glucanases, amylase, hyaluronidase, polygalacturonase (pectinase), dextranase, and cellobiohydrolase.

The composition can further comprise at least one ingredient in a quantity effective to prevent or inhibit inflammation in the ear. This can be a steroid such as hydrocortisone.

The composition can further comprise an antibiotic that is effective in the treatment of *P*. *aeruginosa* in a quantity effective to exert a bactericidal action against P. *aeruginosa.*

The composition can further include lysozyme or lactoferrin. Additionally, the composition can further include at least one peroxidase in a quantity sufficient to exert a bactericidal action. A suitable peroxidase is lactoperoxidase. When the composition includes a peroxidase, the composition can further include at least one substrate that can be converted to an ion with bactericidal properties by the enzymatic action of the peroxidase in a quantity such that an effective concentration of the ion with bactericidal properties is produced by the catalytic action of the peroxidase. When the composition includes a peroxidase, the composition can further include an oxidase in a bactericidally effective quantity, such as glucose oxidase, as well as a substrate for the oxidase, such as glucose when the oxidase is glucose oxidase.

Another aspect of the present invention is a method of treating an ear infection comprising the step of administering a quantity of a composition according to the present invention to a subject with an ear infection in order to treat the infection. The ear infection is typically otitis externa or otitis media and is caused by *Pseudomonas aeruginosa.* The method can further comprise administering an antibiotic that is effective in the treatment of *P. aeruginosa* in a quantity effective to exert a bactericidal action against *P. aeruginosa,* the antibiotic being administered by a route other than the route of administration of the composition. Alternatively, the composition according to the present invention can include an antibiotic that is effective in the treatment of *P. aeruginosa,* in which case the method can further comprise the administration of the same antibiotic or a different antibiotic by a different route.

### ENUMERATED EMBODIMENTS OF THE INVENTION

1. A composition for removal of biofilm in the ear comprising:
   (a) a quantity of at least one enzyme that catalyzes the hydrolysis of a bond that connects two monosaccharides in a polysaccharide or that connects a monosaccharide with a protein molecule in a glycoprotein sufficient to break down biofilm in the ear; and
   (b) a pharmaceutically acceptable carrier suitable for administration into the ear canal.
2. The composition of embodiment 1 wherein the at least one enzyme that catalyzes the hydrolysis of a bond that connects two monosaccharides in a polysaccharide or that connects a monosaccharide with a protein molecule in a glycoprotein is selected from the group consisting of xylanase, β-glucanase, cellulase, α-galactosidase, glucanases, amylase, hyaluronidase, polygalacturonase (pectinase), dextranase, cellobiohydrolase, pullulanase, glycosylceramidase, glucan 1,4-α-glucosidase, oligo-1,6-glucosidase, fucoidanase, glycosylceramidase, glycosylceramidase, thioglucosidase, and glycopeptide N-glycosidase.
3. The composition of embodiment 2 wherein the at least one enzyme that catalyzes the hydrolysis of a bond that connects two monosaccharides in a polysaccharide or that connects a monosaccharide with a protein molecule in a glycoprotein is selected from the group consisting of xylanase, β-glucanase, cellulase, α-galactosidase, glucanases, amylase, hyaluronidase, polygalacturonase (pectinase), dextranase, and cellobiohydrolase.
4. The composition of embodiment 3 wherein the at least one enzyme that catalyzes the hydrolysis of a bond that connects two monosaccharides in a polysaccharide or that connects a monosaccharide with a protein molecule in a glycoprotein is pectinase.
5. The composition of embodiment 3 wherein the at least one enzyme that catalyzes the hydrolysis of a bond that connects two monosaccharides in a polysaccharide or that connects a monosaccharide with a protein molecule in a glycoprotein is dextranase and pectinase.
6. The composition of embodiment 3 wherein the at least one enzyme that catalyzes the hydrolysis of a bond that connects two monosaccharides in a polysaccharide or that connects a monosaccharide with a protein molecule in a glycoprotein is dextranase and xylanase.
7. The composition of embodiment 3 wherein the at least one enzyme that catalyzes the hydrolysis of a bond that connects two monosaccharides in a polysaccharide or that connects a monosaccharide with a protein molecule in a glycoprotein is α-galactosidase and amylase.
8. The composition of embodiment 3 wherein the at least one enzyme that catalyzes the hydrolysis of a bond that connects two monosaccharides in a polysaccharide or that connects a monosaccharide with a protein molecule in a glycoprotein is pectinase and amylase.
9. The composition of embodiment 3 wherein the at least one enzyme that catalyzes the hydrolysis of a bond that connects two monosaccharides in a polysaccharide or that connects a monosaccharide with a protein molecule in a glycoprotein is dextranase, pectinase, and β-D-glucosidase.
10. The composition of embodiment 3 wherein the at least one enzyme that catalyzes the hydrolysis of a bond that connects two monosaccharides in a polysaccharide or that connects a monosaccharide with a protein molecule in a glycoprotein is dextranase, pectinase, and cellulase.
11. The composition of embodiment 3 wherein the at least one enzyme that catalyzes the hydrolysis of a bond that connects two monosaccharides in a polysaccharide or that connects a monosaccharide with a protein molecule in a glycoprotein is dextranase, pectinase, cellulase, amylase, and xylanase.
12. The composition of embodiment 1 wherein the composition further comprises at least one ingredient in a quantity effective to prevent or inhibit inflammation in the ear.
13. The composition of embodiment 12 wherein the at least one ingredient in a quantity effective to prevent or inhibit inflammation in the ear is a steroid.
14. The composition of embodiment 13 wherein the steroid is a steroid selected from the group consisting of hydrocortisone, beclomethasone, budenoside, ciclesonide, flunisolide, fluticasone, methylprednisolone, prednisolone, prednisone, and triamcinolone, and the salts, solvates, analogues, congeners, bioisosteres, hydrolysis products, metabolites, precursors, and prodrugs thereof.
15. The composition of embodiment 14 wherein the steroid is hydrocortisone.
16. The composition of embodiment 1 wherein the pharmaceutically acceptable carrier suitable for administration into the ear canal includes propylene glycol.
17. The composition of embodiment 16 wherein the pharmaceutically acceptable carrier suitable for administration into the ear canal includes propylene glycol and glycerol.
18. The composition of embodiment 17 wherein the pharmaceutically acceptable carrier suitable for administration into the ear canal includes propylene glycol, glycerol, and tripropylene glycol.
19. The composition of embodiment 1 wherein the composition further comprises an antibiotic that is effective in the treatment of P. *aeruginosa* in a quantity effective to exert a bactericidal action against P. *aeruginosa.*
20. The composition of embodiment 19 wherein the antibiotic is selected from the group consisting of amikacin, ticarcillin, piperacillin, mezlocillin, azlocillin, ceftazidime, cefepime, ciprofloxacin, tobramycin, aztreonam, imipenem, and meropenem.
21. The composition of embodiment 1 further including lysozyme in a quantity sufficient to exert a bactericidal action.
22. The composition of embodiment 1 further including lactoferrin in a quantity sufficient to exert a bactericidal action.
23. The composition of embodiment 1 further including at least one peroxidase in a quantity sufficient to exert a bactericidal action.
24. The composition of embodiment 23 wherein the at least one peroxidase is selected from the group consisting of lactoperoxidase, myeloperoxidase, horseradish peroxidase, eosinophil peroxidase, and glutathione peroxidase.
25. The composition of embodiment 24 wherein the at least one peroxidase is selected from the group consisting of lactoperoxidase and myeloperoxidase.
26. The composition of embodiment 25 wherein the at least one peroxidase is lactoperoxidase.
27. The composition of embodiment 23 wherein the composition further includes at least one substrate that can be converted to an ion with bactericidal properties by the enzymatic action of the peroxidase in a quantity such that an effective concentration of the ion with bactericidal properties is produced by the catalytic action of the peroxidase.
28. The composition of embodiment 27 wherein the at least one substrate is an alkali metal salt of thiocyanate, iodate, or chlorate.
29. The composition of embodiment 28 wherein the alkali metal salt is a sodium or potassium salt.
30. The composition of embodiment 29 wherein the alkali metal salt is potassium thiocyanate or potassium iodate.
31. The composition of embodiment 23 wherein the composition further includes a catalase inhibitor.
32. The composition of embodiment 31 wherein the catalase inhibitor is selected from the group consisting of sodium ascorbate, potassium ascorbate, calcium ascorbate, ascorbyl palmitate, and mixtures thereof.
33. The composition of embodiment 32 wherein the composition further includes an iron salt selected from the group consisting of ferrous sulfate, ferrous chloride, and ferrous iodide.
34. The composition of embodiment 33 wherein the iron salt is ferrous sulfate.
35. The composition of embodiment 27 wherein the composition further includes an aminohexose in a quantity effective to increase the yield or accumulation of oxidized anionic biocidal agent.
36. The composition of embodiment 35 wherein the aminohexose is an aminoglucose.
37. The composition of embodiment 36 wherein the aminoglucose is selected from the group consisting of glucosamine, N-acetylglucosamine, and mixtures thereof.
38. The composition of embodiment 23 wherein the composition further includes an oxidase in a bactericidally effective quantity.
39. The composition of embodiment 38 wherein the oxidase is selected from the group consisting of glucose oxidase, galactose oxidase, urate oxidase, choline oxidase, D-amino acid oxidase, D-glutamate oxidase, glycine oxidase, glycolic oxidase, L-sorbose oxidase, alcohol oxidase, and amine oxidase.
40. The composition of embodiment 39 wherein the oxidase is glucose oxidase.
41. The composition of embodiment 38 wherein the composition further includes a substrate for the oxidase in a bactericidally effective quantity.
42. The composition of embodiment 40 wherein the composition further includes glucose as a substrate for the glucose oxidase in a bactericidally effective quantity.
43. The composition of embodiment 1 wherein the composition further includes a thickener to provide the composition with an enzyme immobilizing viscosity which inhibits enzymatic action during processing and in packing.
44. The composition of embodiment 43 wherein the thickener is hydroxypropylcellulose.
45. The composition of embodiment 1 that is aqueous.
46. The composition of embodiment 1 that is non-aqueous.
47. The composition of embodiment 1 that is formulated to treat otitis media.
48. The composition of embodiment 1 that is formulated to treat otitis externa.
49. The composition of embodiment 1 wherein the composition is formulated to treat infection by *Pseudomonas aeruginosa.*
50. The composition of embodiment 1 wherein the composition comprises:
   (a) from about 12.33% to about 18.49% of glycerol;
   (b) from about 71.661% to about 83.986% of propylene glycol;
   (c) from about 0.352% to about 0.528% of hydroxypropylcellulose;
   (d) from about 2.405% to about 3.607% of benzyl alcohol;
   (e) from about 0.0072% to about 0.0108% of pectinase; and
   (f) from about 0.92% to about 1.38% of water.
51. The composition of embodiment 50 wherein the composition comprises:
   (a) about 15.41 % of glycerol;
   (b) about 79.623% of propylene glycol;
   (c) about 0.440% of hydroxypropylcellulose;
   (d) about 3.006% of benzyl alcohol;
   (e) about 0.009% of pectinase; and
   (f) about 1.150% of water.
52. The composition of embodiment 1 wherein the composition comprises:
   (a) from about 28.328% to about 42.492% of glycerol;
   (b) from about 52.761% to about 64.485% of propylene glycol;
   (c) from about 1.152% to about 1.728% of tripropylene glycol;
   (d) from about 2.405% to about 3.607% of benzyl alcohol;
   (e) from about 0.80% to about 1.20% of hydrocortisone;
   (f) from about 0.008% to about 0.012% of pectinase; and
   (g) from about 0.80% to about 1.20% of water.
53. The composition of embodiment 52 wherein the composition comprises:
   (a) about 35.410% of glycerol;
   (b) about 58.623% of propylene glycol;
   (c) about 1.440% of tripropylene glycol;
   (d) about 3.006% of benzyl alcohol;
   (e) about 1.00% of hydrocortisone;
   (f) about 0.010% of pectinase; and
   (g) about 1.00% of water.
54. The composition of embodiment 1 wherein the composition comprises:
   (a) from about 24.328% to about 36.492% of glycerol;
   (b) from about 57.261 % to about 69.985% of propylene glycol;
   (c) from about 1.152% to about 1.728% of tripropylene glycol;
   (d) from about 2.405% to about 3.607% of benzyl alcohol;
   (e) from about 0.008% to about 0.012% of dextranase;
   (f) from about 0.008% to about 0.012% of pectinase; and
   (g) from about 1.201 % to about 1.801 % of water.
55. The composition of embodiment 54 wherein the composition comprises:
   (a) about 30.410% of glycerol;
   (b) about 63.623% propylene glycol;
   (c) about 1.440% of tripropylene glycol;
   (d) about 3.006% of benzyl alcohol;
   (e) about 0.010% of dextranase;
   (f) about 0.010% of pectinase; and
   (g) about 1.501 % of water.
56. The composition of embodiment 1 wherein the composition comprises:
   (a) from about 16.328% to about 24.492% of glycerol;
   (b) from about 63.561% to about 76.506% of propylene glycol;
   (c) from about 0.40% to about 0.60% of hydroxypropylcellulose;
   (d) from about 2.405% to about 3.607% of benzyl alcohol;
   (e) from about 0.008% to about 0.012% of dextranase; and
   (f) from about 4.353% to about 6.529% of water.
57. The composition of embodiment 56 wherein the composition comprises:
   (a) about 20.410% of glycerol;
   (b) about 70.623% of propylene glycol;
   (c) about 0.50% of hydroxypropylcellulose;
   (d) about 3.006% of benzyl alcohol;
   (e) about 0.010% of dextranase; and
   (f) about 5.441 % of water.
58. The composition of embodiment 1 wherein the composition comprises:
   (a) from about 16.328% to about 24.492% of glycerol;
   (b) from about 63.561% to about 76.490% of propylene glycol;
   (c) from about 0.40% to about 0.60% of hydroxypropylcellulose;
   (d) from about 2.405% to about 3.607% of benzyl alcohol;
   (e) from about 0.008% to about 0.012% of dextranase;
   (f) from about 0.008% to about 0.012% of lactoperoxidase;
   (g) from about 0.008% to about 0.012% of pectinase; and
   (h) from about 4.353% to about 6.529% of water.
59. The composition of embodiment 58 wherein the composition comprises:
   (a) about 20.410% of glycerol;
   (b) about 70.623% of propylene glycol;
   (c) about 0.50% of hydroxypropylcellulose;
   (d) about 3.006% of benzyl alcohol;
   (e) about 0.010% of dextranase;
   (f) about 0.010% of lactoperoxidase;
   (g) about 0.010% of pectinase; and
   (h) about 5.441 % of water.
60. The composition of embodiment 1 wherein the composition comprises:
   (a) from about 20.328% to about 30.492% of glycerol;
   (b) from about 59.061% to about 72.185% of propylene glycol;
   (c) from about 0.40% to about 0.60% of hydroxypropylcellulose;
   (d) from about 2.405% to about 3.607% of benzyl alcohol;
   (e) from about 0.008% to about 0.012% of lysozyme;
   (f) from about 0.008% to about 0.012% of lactoferrin;
   (g) from about 0.008% to about 0.012% of lactoperoxidase;
   (h) from about 0.008% to about 0.012% of pectinase; and
   (i) from about 4.353% to about 6.529% of water.
61. The composition of embodiment 60 wherein the composition comprises:
   (a) about 25.410% of glycerol;
   (b) about 65.523% of propylene glycol;
   (c) about 0.50% of hydroxypropylcellulose;
   (d) about 3.006% of benzyl alcohol;
   (e) about 0.010% of lysozyme;
   (f) about 0.010% of lactoferrin;
   (g) about 0.010% of lactoperoxidase;
   (h) about 0.010% of pectinase; and
   (i) about 5.441 % of water.
62. The composition of embodiment 1 wherein the composition comprises:
   (a) from about 16.830% to about 25.246% of glycerol;
   (b) from about 65.518% to about 78.248% of propylene glycol;
   (c) from about 0.412% to about 0.618% of hydroxypropylcellulose;
   (d) from about 0.00824% to about 0.0124% of dextranase;
   (e) from about 0.00824% to about 0.0124% of lactoperoxidase;
   (f) from about 0.00824% to about 0.0124% of pectinase; and
   (g) from about 4.486% to about 6.730% of water.
63. The composition of embodiment 62 wherein the composition comprises:
   (a) about 21.038% of glycerol;
   (b) about 72.798% of propylene glycol;
   (c) about 0.515% of hydroxypropylcellulose;
   (d) about 0.0103% of dextranase;
   (e) about 0.0103% of lactoperoxidase;
   (f) about 0.0103% of pectinase; and
   (g) about 5.608% of water.
64. The composition of embodiment 1 wherein the composition comprises:
   (a) from about 25.201% to about 37.813% of glycerol;
   (b) from about 47.114% to about 57.584% of propylene glycol;
   (c) from about 8.375% to about 12,563% of tripropylene glycol;
   (d) from about 0.00832% to about 0.0125% of dextranase;
   (e) from about 0.00832% to about 0.0125% of lactoperoxidase;
   (f) from about 0.00832% to about 0.0125% of pectinase; and
   (g) from about 4.510% to about 6.766% of water.
65. The composition of embodiment 64 wherein the composition comprises:
   (a) about 31.511% of glycerol;
   (b) about 52.349% of propylene glycol;
   (c) about 10.469% of tripropylene glycol;
   (4) about 0.0104% of dextranase;
   (5) about 0.0104% of lactoperoxidase;
   (6) about 0.0104% of pectinase; and
   (7) about 5.638% of water.
66. The composition of embodiment 1 wherein the composition comprises:
   (a) from about 16.919% to about 25.379% of glycerol;
   (b) from about 56.440% to about 68.982% of propylene glycol;
   (c) from about 8.383% to about 12.575% of tripropylene glycol;
   (d) from about 0.00832% to about 0.0125% of dextranase;
   (e) from 0.00832% to about 0.0125% of xylanase; and
   (f) from about 4.510% to about 6.766% of water.
67. The composition of embodiment 66 wherein the composition comprises:
   (a) about 21.149% of glycerol;
   (b) about 62.711% of propylene glycol;
   (c) about 10.479% of tripropylene glycol;
   (d) about 0.104% of dextranase;
   (e) about 0.104% of xylanase; and
   (f) about 5.638% of water.
68. The composition of embodiment 1 wherein the composition comprises:
   (a) from about 12.348% to about 18.522% of glycerol;
   (b) from about 73.051% to about 84.927% of propylene glycol;
   (c) from about 0.460% to about 0.690% of hydroxypropylcellulose;
   (d) from about 0.0092% to about 0.0138% of α-galactosidase;
   (e) from about 0.0092% to about 0.0138% of amylase; and
   (f) from about 2.247% to about 3.371 % of water.
69. The composition of embodiment 68 wherein the composition comprises:
   (a) about 15.435% of glycerol;
   (b) about 81.168% of propylene glycol;
   (c) about 0.575% of hydroxypropylcellulose;
   (d) about 0.0115% of α-galactosidase;
   (e) about 0.0115% of amylase; and
   (f) about 2.809% of water.
70. The composition of embodiment 1 wherein the composition comprises:
   (a) from about 14.596% to about 21.894% of glycerol;
   (b) from about 69.818% to about 82.069% of propylene glycol;
   (c) from about 0.00944% to about 0.0141% of lactoperoxidase;
   (d) from about 0.0474% to about 0.0710% of pectinase;
   (e) from about 0.0190% to about 0.0284% of amylase; and
   (f) from about 3.259% to about 4.889% of water.
71. The composition of embodiment 70 wherein the composition comprises:
   (a) about 18.245% of glycerol;
   (b) about 77.576% of propylene glycol;
   (c) about 0.0118% of lactoperoxidase;
   (d) about 0.0592% of pectinase;
   (e) about 0.0237% of amylase; and
   (f) about 4.074% of water.
72. The composition of embodiment 1 wherein the composition comprises:
   (a) from about 25.204% to about 37.806% of glycerol;
   (b) from about 47.105% to about 57.572% of propylene glycol;
   (c) from about 8.374% to about 12.560% of tripropylene glycol;
   (d) from about 0.00832% to about 0.0125% of dextranase;
   (e) from about 0.0166% to about 0.0248% of lactoperoxidase;
   (f) from about 0.00832% to about 0.0125% of pectinase;
   (g) from about 0.00832% to about 0.0125% of potassium thiocyanate; and
   (h) from about 4.510% to about 6.764% of water.
73. The composition of embodiment 72 wherein the composition comprises:
   (a) about 31.505% of glycerol;
   (b) about 52.339% of propylene glycol;
   (c) about 10.467% of tripropylene glycol;
   (d) about 0.0104% of dextranase;
   (e) about 0.0207% of lactoperoxidase;
   (f) about 0.0104% of pectinase;
   (g) about 0.0104% of potassium thiocyanate; and
   (h) about 5.637% of water.
74. The composition of embodiment 1 wherein the composition comprises:
   (a) from about 12.846% to about 19.268% of glycerol;
   (b) from about 75.510% to about 87.112% of propylene glycol;
   (c) from about 0.00832% to about 0.0125% of dextranase;
   (d) from about 0.0166% to about 0.0250% of lactoperoxidase;
   (e) from about 0.00832% to about 0.0125% of pectinase; and
   (f) from about 0.00832% to about 0.0125% of potassium thiocyanate.
75. The composition of embodiment 74 wherein the composition comprises:
   (a) about 16.057% of glycerol;
   (b) about 83.901% of propylene glycol;
   (c) about 0.0104% of dextranase;
   (d) about 0.0208% of lactoperoxidase;
   (e) about 0.0104% of pectinase; and
   (f) about 0.0104% of potassium thiocyanate.
76. The composition of embodiment 1 wherein the composition comprises:
   (a) from about 12.834% to about 19.250% of glycerol;
   (b) from about 75.439% to about 87.100% of propylene glycol;
   (c) from about 0.00832% to about 0.0125% of dextranase;
   (d) from about 0.0166% to about 0.0250% of lactoperoxidase;
   (e) from about 0.0166% to about 0.0250% of pectinase;
   (f) from about 0.0166% to about 0.0250% of lysozyme; and
   (g) from about 0.00832% to about 0.0125% of potassium iodate.
77. The composition of embodiment 76 wherein the composition comprises:
   (a) about 16.042% of glycerol;
   (b) about 83.821 % of propylene glycol;
   (c) about 0.0104% of dextranase;
   (d) about 0.0208% of lactoperoxidase;
   (e) about 0.0208% of pectinase;
   (f) about 0.0208% of lysozyme; and
   (g) about 0.0104% of potassium iodate.
78. The composition of embodiment 1 wherein the composition comprises:
   (a) from about 8.670% to about 13.004% of glycerol;
   (b) from about 75.439% to about 87.091% of propylene glycol;
   (c) from about 4.164% to about 6.246% of tripropylene glycol;
   (d) from about 0.00832% to about 0.0125% of dextranase;
   (e) from about 0.0166% to about 0.0250% of lactoperoxidase;
   (f) from about 0.0166% to about 0.0250% of pectinase;
   (g) from about 0.0166% to about 0.0250% of lysozyme;
   (h) from about 0.00832% to about 0.0125% of lactoferrin; and
   (i) from about 0.00832% to about 0.0125% of potassium iodate.
79. The composition of embodiment 78 wherein the composition comprises:
   (a) about 10.847% of glycerol;
   (b) about 83.821% of propylene glycol;
   (c) about 5.205% of tripropylene glycol;
   (d) about 0.0104% of dextranase;
   (e) about 0.0208% of lactoperoxidase;
   (f) about 0.0208% of pectinase;
   (g) about 0.0208% of lysozyme;
   (h) about 0.0104% of lactoferrin; and
   (i) about 0.0104% of potassium iodate.
80. The composition of embodiment 1 wherein the composition comprises:
   (a) from about 8.678% to about 13.016% of glycerol;
   (b) from about 75.512% to about 87.104% of propylene glycol;
   (c) from about 4.168% to about 6.252% of tripropylene glycol;
   (d) from about 0.00832% to about 0.0125% of dextranase;
   (e) from about 0.0166% to about 0.0250% of lactoperoxidase;
   (f) from about 0.00832% to about 0.0125% of pectinase;
   (g) from about 0.00832% to about 0.0125% of β-D-glucosidase;
   (h) from about 0.00832% to about 0.0125% of potassium iodate.
81. The composition of embodiment 80 wherein the composition comprises:
   (a) about 10.847% of glycerol;
   (b) about 83.902% of propylene glycol;
   (c) about 5.210% of tripropylene glycol;
   (d) about 0.0104% of dextranase;
   (e) about 0.0208% of lactoperoxidase;
   (f) about 0.0104% of pectinase;
   (g) about 0.0104% of β-D-glucosidase; and
   (h) about 0.0104% of potassium iodate.
82. The composition of embodiment 1 wherein the composition comprises:
   (a) from about 17.014% to about 25.520% of glycerol;
   (b) from about 66.134% to about 78.768% of propylene glycol;
   (c) from about 4.168% to about 6.252% of tripropylene glycol;
   (d) from about 0.00832% to about 0.0125% of dextranase;
   (e) from about 0.0166% to about 0.0250% of lactoperoxidase;
   (f) from about 0.00832% to about 0.0125% of pectinase;
   (g) from about 0.00832% to about 0.0125% of cellulase; and
   (h) from about 0.00832% to about 0.0125% of potassium thiocyanate.
83. The composition of embodiment 82 wherein the composition comprises:
   (a) about 21.267% of glycerol;
   (b) about 73.482% of propylene glycol;
   (c) about 5.210% of tripropylene glycol;
   (d) about 0.0104% of dextranase;
   (e) about 0.0208% of lactoperoxidase;
   (f) about 0.0104% of pectinase;
   (g) about 0.0104% of cellulase; and
   (h) about 0.0104% of potassium thiocyanate.
84. The composition of embodiment 1 wherein the composition comprises:
   (a) from about 16.998% to about 25.496% of glycerol;
   (b) from about 66.070% to about 78.763% of propylene glycol;
   (c) from about 4.164% to about 6.246% of tripropylene glycol;
   (d) from about 0.00832% to about 0.0125% of dextranase;
   (e) from about 0.0166% to about 0.0250% of lactoperoxidase;
   (f) from about 0.00832% to about 0.0125% of pectinase;
   (g) from about 0.00832% to about 0.0125% of cellulase;
   (h) from about 0.0166% to about 0.0250% of amylase;
   (i) from about 0.00832% to about 0.0125% of xylanase; and
   (j) from about 0.00832% to about 0.0125% of potassium thiocyanate.
85. The composition of embodiment 84 wherein the composition comprises:
   (a) about 21.247% of glycerol;
   (b) about 73.411 % of propylene glycol;
   (c) about 5.205% of tripropylene glycol;
   (d) about 0.0104% of dextranase;
   (e) about 0.0208% of lactoperoxidase;
   (f) about 0.0104% of pectinase;
   (g) about 0.0104% of cellulase;
   (h) about 0.0208% of amylase;
   (i) about 0.0104% of xylanase; and
   (j) about 0.0104% of potassium thiocyanate.
86. The composition of embodiment 1 wherein the composition comprises:
   (a) from about 19.311% to about 28.967% of glycerol;
   (b) from about 65.497% to about 78.212% of propylene glycol;
   (c) from about 2.194% to about 3.296% of benzyl alcohol;
   (d) from about 0.220% to about 0.330% of β-D-glucose;
   (e) from about 0.00731 % to about 0.0110% of dextranase;
   (f) from about 0.00658% to about 0.00988% of lactoperoxidase;
   (g) from about 0.00585% to about 0.00877% of glucose oxidase;
   (h) from about 0.00658% to about 0.00988% of pectinase;
   (i) from about 0.00731 % to about 0.0110% of dextranase;
   (j) from about 0.0146% to about 0.0220% of amylase;
   (k) from about 0.00731% to about 0.0110% of xylanase; and
   (l) from about 0.00731% to about 0.0110% of potassium iodate.
87. The composition of embodiment 86 wherein the composition comprises:
   (a) about 24.139% of glycerol;
   (b) about 72.775% of propylene glycol;
   (c) about 2.747% of benzyl alcohol;
   (d) about 0.275% of β-D-glucose;
   (e) about 0.00914% of dextranase;
   (f) about 0.00823% of lactoperoxidase;
   (g) about 0.00731 % of glucose oxidase;
   (h) about 0.00823% of pectinase;
   (i) about 0.00914% of cellulase;
   (j) about 0.0183% of amylase;
   (k) about 0.00914% of xylanase; and
   (l) about 0.00914% of potassium iodate.
88. The composition of embodiment 1 wherein the composition comprises:
   (a) from about 19.142% to about 28.712% of glycerol;
   (b) from about 64.924% to about 77.702% of propylene glycol;
   (c) from about 2.178% to about 3.268% of benzyl alcohol;
   (d) from about 0.725% to about 1.087% of hydrocortisone;
   (e) from about 0.218% to about 0.328% of β-D-glucose;
   (f) from about 0.00725% to about 0.0109% of dextranase;
   (g) from about 0.00652% to about 0.00978% of lactoperoxidase;
   (h) from about 0.00580% to about 0.00870% of glucose oxidase;
   (i) from about 0.00725% to about 0.0109% of pectinase; and
   (j) from about 0.00725% to about 0.0109% of potassium iodate.
89. The composition of embodiment 88 wherein the composition comprises:
   (a) about 23.927% of glycerol;
   (b) about 72.138% of propylene glycol;
   (c) about 2.723% of benzyl alcohol;
   (d) about 0.906% of hydrocortisone;
   (e) about 0.273% of β-D-glucose;
   (f) about 0.00906% of dextranase;
   (g) about 0.00815% of lactoperoxidase;
   (h) about 0.00725% of glucose oxidase;
   (i) about 0.00906% of pectinase; and
   (j) about 0.00906% of potassium iodate.
90. The composition of embodiment 1 wherein the composition comprises:
   (a) from about 28.328% to about 42.492% of glycerol;
   (b) from about 52.761% to about 64.485% of propylene glycol;
   (c) from about 1.152% to about 1.728% of hydroxypropylcellulose;
   (d) from about 2.405% to about 3.607% of benzyl alcohol;
   (e) from about 0.120% to about 0.180% of water;
   (f) from about 0.800% to about 1.200% of hydrocortisone;
   (g) from about 0.241% to about 0.361% of β-D-glucose;
   (h) from about 0.0064% to about 0.0096% of lactoperoxidase;
   (i) from about 0.0008% to about 0.0012% of glucose oxidase;
   (j) from about 0.0064% to about 0.0096% of lactoferrin;
   (k) from about 0.0064% to about 0.0096% of lysozyme;
   (l) from about 0.0080% to about 0.0120% of pectinase; and
   (m) from about 0.028% to about 0.042% of potassium iodate.
91. The composition of embodiment 90 wherein the composition comprises:
   (a) about 35.410% of glycerol;
   (b) about 58.623% of propylene glycol;
   (c) about 1.440% of hydroxypropylcellulose;
   (d) about 3.006% of benzyl alcohol;
   (e) about 0.150% of water;
   (f) about 1.000% of hydrocortisone;
   (g) about 0.301 % of β-D-glucose;
   (h) about 0.008% of lactoperoxidase;
   (i) about 0.001 % of glucose oxidase;
   (j) about 0.008% of lactoferrin;
   (k) about 0.008% of lysozyme;
   (l) about 0.010% of pectinase; and
   (m) about 0.035% of potassium iodate.
92. The composition of embodiment 1 wherein the composition comprises:
   (a) from about 28.328% to about 42.492% of glycerol;
   (b) from about 52.761% to about 64.485% of propylene glycol;
   (c) from about 1.152% to about 1.728% of hydroxypropylcellulose;
   (d) from about 2.405% to about 3.607% of benzyl alcohol;
   (e) from about 0.120% to about 0.180% of water;
   (f) from about 0.800% to about 1.200% of hydrocortisone;
   (g) from about 0.241 % to about 0.361 % of β-D-glucose;
   (h) from about 0.0064% to about 0.0096% of lactoperoxidase;
   (i) from about 0.0008% to about 0.0012% of glucose oxidase;
   (j) from about 0.0064% to about 0.0096% of lactoferrin;
   (k) from about 0.0064% to about 0.0096% of lysozyme;
   (l) rom about 0.0080% to about 0.0120% of pectinase; and
   (m) from about 0.028% to about 0.042% of potassium thiocyanate.
93. The composition of embodiment 92 wherein the composition comprises:
   (a) about 35.410% of glycerol;
   (b) about 58.623% of propylene glycol;
   (c) about 1.440% of hydroxypropylcellulose;
   (d) about 3.006% of benzyl alcohol;
   (e) about 0.150% of water;
   (f) about 1.000% of hydrocortisone;
   (g) about 0.301 % of β-D-glucose;
   (h) about 0.008% of lactoperoxidase;
   (i) about 0.001 % of glucose oxidase;
   (j) about 0.008% of lactoferrin;
   (k) about 0.008% of lysozyme;
   (l) about 0.010% of pectinase; and
   (m) about 0.035% of potassium thiocyanate.
94. The composition of embodiment 1 wherein the composition comprises:
   (a) from about 28.328% to about 42.492% of glycerol;
   (b) from about 53.652% to about 65.574% of propylene glycol;
   (c) from about 1.152% to about 1.728% of hydroxypropylcellulose;
   (d) from about 2.405% to about 3.607% of benzyl alcohol;
   (e) from about 0.120% to about 0.180% of water;
   (f) from about 0.241 % to about 0.361 % of β-D-glucose;
   (g) from about 0.0064% to about 0.0096% of lactoperoxidase;
   (h) from about 0.0008% to about 0.0012% of glucose oxidase;
   (i) from about 0.0064% to about 0.0096% of lactoferrin;
   (j) from about 0.0064% to about 0.0096% of lysozyme;
   (k) from about 0.0080% to about 0.0120% of pectinase;
   (l) from about 0.0080% to about 0.0120% of dextranase; and
   (m) from about 0.028% to about 0.042% of potassium thiocyanate.
95. The composition of embodiment 94 wherein the composition comprises:
   (a) about 35.410% of glycerol;
   (b) about 59.613% of propylene glycol;
   (c) about 1.440% of hydroxypropylcellulose;
   (d) about 3.006% of benzyl alcohol;
   (e) about 0.150% of water;
   (f) about 0.301% of β-D-glucose;
   (g) about 0.008% of lactoperoxidase;
   (h) about 0.001 % of glucose oxidase;
   (i) about 0.008% of lactoferrin;
   (j) about 0.008% of lysozyme;
   (k) about 0.010% of pectinase;
   (l) about 0.010% of dextranase;
   (m) about 0.035% of potassium thiocyanate.
96. The composition of embodiment 1 wherein the composition comprises:
   (a) from about 61.2% to about 74.4% of squalene;
   (b) from about 6.8% to about 10.2% of propylene glycol;
   (c) from about 16.32% to about 24.48% of glycerol;
   (d) from about 2.4% to about 3.6% of water;
   (e) from about 0.012% to about 0.018% of lactoperoxidase;
   (f) from about 0.008% to about 0.012% of lactoferrin;
   (g) from about 0.008% to about 0.012% of lysozyme;
   (h) from about 0.048% to about 0.072% of potassium thiocyanate; and
   (i) from about 0.0096% to about 0.0144% of pectinase.
97. The composition of embodiment 96 wherein the composition comprises:
   (a) about 68% of squalene;
   (b) about 8.5% of propylene glycol;
   (c) about 20.4% of glycerol;
   (d) about 3% of water;
   (e) about 0.015% of lactoperoxidase;
   (f) about 0.010% of lactoferrin;
   (g) about 0.010% of lysozyme;
   (h) about 0.060% of potassium thiocyanate; and
   (i) about 0.012% of pectinase.
98. The composition of embodiment 1 wherein the composition comprises:
   (a) from about 38.16% to about 46.64% of squalene;
   (b) from about 5.2% to about 7.8% of propylene glycol;
   (c) from about 43.2% to about 52;8% of glycerol;
   (d) from about 2.4% to about 3.6% of water;
   (e) from about 0.012% to about 0.018% of lactoperoxidase;
   (f) from about 0.008% to about 0.012% of lactoferrin;
   (g) from about 0.008% to about 0.012% of lysozyme;
   (h) from about 0.032% to about 0.048% of potassium iodate; and
   (i) from about 0.0096% to about 0.0144% of amylase.
99. The formulation of embodiment 98 wherein the composition comprises:
   (a) about 42.4% of squalene;
   (b) about 6.5% of propylene glycol;
   (c) about 48% of glycerol;
   (d) about 3% of water;
   (e) about 0.015% of lactoperoxidase;
   (f) about 0.010% of lactoferrin;
   (g) about 0.010% of lysozyme;
   (h) about 0.04% of potassium iodate; and
   (i) about 0.012% of amylase.
100. A method of treating an ear infection comprising the step of administering a quantity of the composition of embodiment 1 to a subject with an ear infection in order to treat the infection.
101. The method of embodiment 100 wherein the ear infection is otitis media.
102. The method of embodiment 100 wherein the ear infection is otitis externa.
103. The method of embodiment 100 wherein the ear infection is caused by *Pseudomonas aeruginosa.*
104. The method of embodiment 103 wherein the method further comprises administering an antibiotic that is effective in the treatment of P. *aeruginosa* in a quantity effective to exert a bactericidal action against P. *aeruginosa,* the antibiotic being administered by a route other than the route of administration of the composition.
105. The method of embodiment 104 wherein the route other than the route of administration of the composition is oral.
106. The method of embodiment 104 wherein the route other than the route of administration of the composition is parenteral.
107. The method of embodiment 106 wherein the route other than the route of administration of the composition is intramuscular.
108. The method of embodiment 103 wherein the composition further comprises an antibiotic that is effective in the treatment of P. *aeruginosa* in a quantity effective to exert a bactericidal action against *P. aeruginosa.*
109. The method of embodiment 108 wherein the method further comprises administering an antibiotic that is effective in the treatment of *P. aeruginosa* in a quantity effective to exert a bactericidal action against *P. aeruginosa,* the antibiotic being administered by a route other than the route of administration of the composition.
110. The method of embodiment 109 wherein the antibiotic that is administered by the route other than the route of administration of the composition is the same as the antibiotic included in the composition.
111. The method of embodiment 109 wherein the antibiotic that is administered by the route other than the route of administration of the composition is different than the antibiotic included in the composition.

### DETAILED DESCRIPTION OF THE INVENTION

Antibiotics are the primary treatment for ear infection but, as discussed earlier, only kill free-floating bacteria. It is difficult if not impossible, for antibiotics to kill bacteria embedded in a biofilm.

By the application of a biofilm-dissolving enzyme system first to the ear or together with an antibiotic, the antibiotic is made much more effective.

In general, a biofilm-dissolving enzyme suitable for use in methods according to the present invention is an enzyme that catalyzes the hydrolysis of a bond that connects two monosaccharides in a polysaccharide or that connects a monosaccharide with a protein molecule in a glycoprotein. These enzymes are referred to herein as "glycoside linkage-hydrolyzing enzymes."

Biofilm-dissolving enzymes suitable for use in compositions and methods according to the present invention include, but are not limited to, xylanase, β-glucanase, cellulase, α-galactosidase, glucanases, amylase, hyaluronidase, polygalacturonase (pectinase), dextranase, and cellobiohydrolase. Other hydrolytic enzymes that are capable of dissolving a bond that connects two monosaccharides in a polysaccharide or that connects a monosaccharide with a protein molecule in a glycoprotein can also be used, including, but not limited to, pullulanase, glycosylceramidase, glucan 1,4-α-glucosidase, oligo-1,6-glucosidase, fucoidanase, glycosylceramidase, glycosylceramidase, thioglucosidase, and glycopeptide N-glycosidase, as well as other enzymes.

Xylanase (EC 3.2.1.8), more precisely, endo-1,4-β-xylanase, is the name given to a class of enzymes that degrade the linear polysaccharide β-1,4-xylan into the monosaccharide xylose. Xylanase catalyzes the endohydrolysis of 1,4-β-D-xylosidic linkages in xylans. Xylanase is produced by many microorganisms, including *Thermomyces lanuginosus.* Information on xylanase is available at http://www.brenda.uni-koeln.de/php/result flat. php4?ecno=3.2.1.8.

β-glucanase (EC 3.2.1.6), more precisely, endo-1,3(4)- β-glucanase, is an enzyme that catalyzes the endohydrolysis of 1,3- or 1,4-linkages in β-D-glucans when the D-glucose residue whose reducing group is involved in the linkage to be hydrolyzed is itself substituted at C-3. Many sources of β-glucanase are known, particularly from plants and fungi, such as *Candida utilis* and *Saccharomyces cerevisiae.* Information on β-glucanase is available at http://www.brenda.uni-koeln.de/php/result flat.php4?ecno=3.2.1.6.

Cellulase (EC 3.2.1.4) is an enzyme that catalyzes the endohydrolysis of 1,4-β-D-glucosidic linkages in cellulose, lichenin and cereal β-D-glucans. Sources for cellulase include *Aspergillus niger, Clostridium thermocellum,* and *Cellulomonas fimi.* Information on cellulase is available at http://www.brenda.uni-koeln.de/php/result_flat.php4?ecno=3.2.1.4.

α-galactosidase (EC 3.2.1.22) is an enzyme that catalyzes the hydrolysis of terminal, non-reducing alpha-D-galactose residues in α-D-galactosides, including galactose oligosaccharides, galactomannans and galactohydrolase. Sources of α-galactosidase include *A*. *niger, E. coli, Glycine max* (soybean), and *Lactobacillus plantarum.* Information on α-galactosidase is available at http://www.brenda. uni-koeln.de/php/result flat.php4?ecno=3.2.1.22.

Glucanase, or 1,3-β-D-glucosidase (EC 3.2.1.39), is an enzyme that catalyzes the hydrolysis of 1,3-β-D-glucosidic linkages in 1,3-β-D-glucans. Sources of glucanase include *Arabidopsis thaliana, C. thermocellum, Hordeum vulgare,* and *Oryza sativa.* Information on glucanase is available at http://www.brenda.uni-koeln.de/php/result flat.php4?ecno=3.2.1.39.

Amylase, more precisely α-amylase (EC 3.2.1.1) or β-amylase (EC 3.2.1.2), is a class of enzymes that hydrolyzes amylose, a component of starch. The enzyme α-amylase catalyzes the endohydrolysis of 1,4-α-D-glucosidic linkages in polysaccharides containing three or more 1,4-α-linked D-glucose units. Sources of α-amylase include *A. niger, Aspergillus oryzae, Bacillus licheniformis,* and *Bacillus stearothermophilus.* The enzyme β-amylase catalyzes the hydrolysis of 1,4-β-D-glucosidic linkages in polysaccharides so as to remove successive maltose units from the non-reducing ends of the chains. Sources of β-amylase include *H. vulgare* and *Bacillus cereus.* Information on α-amylase is available at http://www.brenda.uni-koeln.de/php/result flatphp4?ecno=3.2.1.1. Information on β-amylase is available at http://www.brenda.uni-koeln.de/php/result flat.php4?ecno=3.2.1.2.

Hyaluronidase, more precisely hyaluronate lyase (EC 4.2.2.1), catalyzes the cleavage of hyaluronate chains at a β-D-GalNAc-(1-4)-β-D-GlcA bond, ultimately breaking the polysaccharide down to 3-(4-deoxy-β-D-gluc-4-enuronosyl)-N-acetyl-D-glucosamine. Sources for hyaluronidase include *Candida albicans* and *Streptomyces griseus.* Information on hyaluronidase is available at http://www.brenda.uni-koeln.de/php/result flat.php4?ecno=4.2.2*.*1.

Polygalacturonase, also known as pectinase, and whose systematic name is poly(1,4-α-D-galacturonide) glycanohydrolase (EC 3.2.1.15), catalyzes the hydrolysis of 1,4-α-D-galactosiduronic linkages in pectate and other galacturonans. Sources for polygalacturonase include *A. niger* and G. *max.* Information on polygalacturonase is available at http://www.brenda.uni-koeln.de/php/result flat.php4?ecno=3.2.1.15. A suitable preparation of pectinase is marketed by Novo Nordisk as Pectinex Ultra SPL™.

Dextranase, whose systematic name is 1,6-α-D-glucan 6-glucanohydrolase, catalyzes the endohydrolysis of 1,6-α-D-glucosidic linkages in dextran. Sources of dextranase include *Penicillum funiculosum* and *Avena sativa.* Information on dextranase is available at http://www.brenda.uni-koeln.de/php/result flat.php4?ecno=3.2.1.11.

Cellobiohydrolase, also known as cellulase, and whose systematic name is 1,4-(1,3;1,4)-β-D-glucan 4-glucanohydrolase (EC 3.2.1.4), catalyzes the endohydrolysis of 1,4-β-D-glucosidic linkages in cellulose, lichenin and cereal β-D-glucans. Sources of cellobiohydrolase include *A. niger* and *Clostridium cellulolyticum.* Information on cellobiohydrolase is available at http://www.brenda.uni-koeln.de/php/result flat.php4?ecno=3.2.1.4.

One or more of these enzymes is included in a composition according to the present invention, together with a pharmaceutically acceptable carrier suitable for administration into the ear canal. Accordingly, one embodiment of the present invention is a composition for removal of biofilm in the ear comprising:
(1) a quantity of at least one enzyme that catalyzes the hydrolysis of a bond that connects two monosaccharides in a polysaccharide or that connects a monosaccharide with a protein molecule in a glycoprotein sufficient to break down biofilm in the ear; and
(2) a pharmaceutically acceptable carrier suitable for administration into the ear canal.

The pharmaceutically acceptable carrier suitable for administration into the ear canal can include buffers, ingredients to control the viscosity of the composition, preservatives, and other conventional ingredients as known in the art. Examples of specific ingredients included in the compositions are provided below in Formulation 1 through Formulation 23.

Typically, in compositions according to the present invention, the pharmaceutically acceptable carrier suitable for administration into the ear canal includes propylene glycol. Preferably, the pharmaceutically acceptable carrier suitable for administration into the ear canal includes propylene glycol and glycerol. In another preferred alternative, the pharmaceutically acceptable carrier suitable for administration into the ear canal includes propylene glycol, glycerol, and tripropylene glycol.

Compositions according to the present invention can further include at least one ingredient in a quantity effective to prevent or inhibit inflammation in the ear. A suitable ingredient is a steroid, such as, but not limited to, a steroid selected from the group consisting of hydrocortisone, beclomethasone, budenoside, ciclesonide, flunisolide, fluticasone, methylprednisolone, prednisolone, prednisone, and triamcinolone, and the salts, solvates, analogues, congeners, bioisosteres, hydrolysis products, metabolites, precursors, and prodrugs thereof. A preferred steroid is hydrocortisone.

Compositions according to the present invention can further include an antibiotic that is effective in the treatment of *P. aeruginosa* in a quantity effective to exert a bactericidal action against *P. aeruginosa.* The antibiotic included in a composition according to the present invention can be, for example, amikacin; a broad-spectrum penicillin such as, but not limited to, ticarcillin, piperacillin, mezlocillin, or azlocillin; ceftazidime; cefepime; ciprofloxacin; tobramycin; aztreonam; imipenem; or meropenem. Alternatively, an antibiotic such as the antibiotics recited above can be administered separately to promote killing of the bacteria in the biofilm. If administered separately, the antibiotic can be administered topically or systemically.

Compositions according to the present invention can further include a quantity of at least one additional antibacterial enzyme that is selected from the group consisting of lysozyme, lactoferrin, and a peroxidase in a quantity sufficient to exert a bactericidal action. Typically, the peroxidase is selected from the group consisting of lactoperoxidase, myeloperoxidase, horseradish peroxidase, eosinophil peroxidase, and glutathione peroxidase. Preferably, the peroxidase is selected from the group consisting of lactoperoxidase and myeloperoxidase. More preferably, the peroxidase is lactoperoxidase. Lactoperoxidase is a glycoprotein which, in one commercial embodiment, is a lyophilized powder derived from milk. This commercial peroxidase has an activity of 80 IU/mg and a projected molecular weight of 93,000 for L-tyrosine iodination. The physicochemical properties reported for lactoperoxidase include a molecular weight of 78,000, a partial specific volume, reflective of the amino acid composition, of 0.74, and the presence of 1.0 mole of heme per mole of lactoperoxidase.

If the composition includes a peroxidase enzyme such as lactoperoxidase, myeloperoxidase, horseradish peroxidase, and eosinophil peroxidase, or glutathione peroxidase, the composition can further include at least one substrate that can be converted to an ion with bactericidal properties by the enzymatic action of the peroxidase enzyme. The substrate is present in a quantity such that an effective concentration of the ion with bactericidal properties is produced by the catalytic action of the peroxidase enzyme. Suitable substrates include, but are not limited to, alkali metal salts of anions such as thiocyanate, iodate, or chlorate. The alkali metal salt is typically a sodium or potassium salt, although other alkali metal salts such as lithium or cesium can alternatively be used. The peroxidase enzyme catalyzes the conversion of thiocyanate into hypothiocyanite (⁻OSCN), molecular oxygen (O₂), and water. The peroxidase enzyme similarly catalyzes the conversion of iodate or chlorate to hypoiodite or hypochlorite. These anions possess bactericidal activity.

In an alternative composition according to the present invention that includes a peroxidase, a catalase inhibitor is further included. The effectiveness of the peroxidase enzyme can be affected by the presence of catalase, which is present in many tissues. Catalase competes with peroxidase for hydrogen peroxide. In order to reduce the loss of hydrogen peroxide through the presence of catalase, an effective amount of an enzymatic inhibitor that is specific for catalase can be advantageously incorporated into a composition according to the present invention. Suitable enzymatic inhibitors specific for catalase include, but are not limited to ascorbic salts such as sodium ascorbate, potassium ascorbate, calcium ascorbate, ascorbyl palmitate, or mixtures thereof, and can be included in a composition according to the invention. An effective concentration of ascorbic salt in compositions according to the present invention is from about 1 × 10⁻⁶ to about 1 × 10⁻⁴ millimole per gram of composition. Iron salts such as ferrous sulfate, ferrous chloride, or ferrous iodide can also be incorporated into a composition according to the present invention as a potentiator for the ascorbic salt in its role as catalase inhibitor. A particularly preferred iron salt is ferrous sulfate.

Compositions according to the present invention that include a peroxidase enzyme and the at least one substrate that can be converted to an ion with bactericidal properties by the enzymatic action of the peroxidase enzyme can also advantageously be formulated with an aminohexose in order to increase the yield or accumulation of oxidized anionic biocidal agent, the quantity of the aminohexose being effective to increase the yield or accumulation of oxidized anionic biocidal agent. Typically, the aminohexose is an aminoglucose, but other aminohexoses such as aminogalactose can alternatively be used. Typically, the aminoglucose is selected from the group consisting of glucosamine, N-acetylglucosamine, and mixtures thereof. The aminoglucose is typically present in the composition in a concentration of from about 0.0001 millimole to about 0.002 millimole per gram of composition. Preferably, the aminoglucose is present in the composition in a concentration of from about 0.0003 millimole to about 0.001 millimole per gram of composition.

Compositions according to the present invention that include a peroxidase can further include an oxidase in a bactericidally effective quantity and, optionally, a substrate for the oxidase in a bactericidally effective quantity. The oxidase oxidizes the substrate and produces hydrogen peroxide, which is then used as a substrate by the peroxidase if present. The use of an oxidase is only required if a peroxidase is also present.

The oxidoreductase enzyme is typically selected from the group consisting of glucose oxidase, galactose oxidase, urate oxidase, choline oxidase, D-amino acid oxidase, D-glutamate oxidase, glycine oxidase, glycolic oxidase, L-sorbose oxidase, alcohol oxidase, and amine oxidase. Other enzymes can alternatively be used, such as nitroethane oxidase, D-aspartate oxidase, L-aminoacid oxidase, pyridoxamine phosphate oxidase, ethanolamine oxidase, pyruvateoxidase, oxalate oxidase, hexose oxidase, cholesterol oxidase, aryl alcohol-,oxidase, pyridoxine 4-oxidase, dehydroorotate oxidase, lathosterol oxidase, sarcosine oxidase, N-methylaminoacid oxidase, N⁶-methyllysine oxidase, 6-hydroxy-L-nicotine oxidase, 6-hydroxy-D-nicotine oxidase, 3-hydroxyanthranilate oxidase, aldehyde oxidase, and xanthine oxidase, as described in U.S. Patent No. 4,340,448 to Schiller et al., incorporated herein by this reference.

For these enzymes, glucose oxidase catalyzes the reaction of β-D-glucose, water, and oxygen to produce hydrogen peroxide and gluconic acid. Galactose oxidase catalyzes the reaction of D-galactose and oxygen to produce hydrogen peroxide and D-galacto-hexodialdose. Urate oxidase catalyzes the reaction of uric acid, water, and oxygen to produce hydrogen peroxide, allantoin, and carbon dioxide. Choline oxidase catalyzes the reaction of choline and oxygen to produce hydrogen peroxide and betaine aldehyde. D-amino acid oxidase catalyzes the reaction of D-amino acids such as D-proline, D-methionine, D-isoleucine, D-alanine, D-valine, or D-phenylalanine with water and oxygen to produce hydrogen peroxide, ammonia, and the α-keto acid corresponding to the D-amino acid being oxidized. D-glutamate oxidase catalyzes the reaction of D-glutamic acid, water, and oxygen to produce hydrogen peroxide, ammonia, and 2-ketoglutarate. Glycine oxidase catalyzes the reaction of glycine, water, and oxygen to produce hydrogen peroxide, ammonia, and glyoxylic acid. Glycolic acid oxidase (also known as 2-hydroxyacid oxidase) catalyzes the reaction of glycolic acid and oxygen to produce 2-ketoacetic acid and hydrogen peroxide. L-sorbose oxidase catalyzes the reaction of L-sorbose and oxygen to produce 5-dehydro-D-fructose and hydrogen peroxide. Alcohol oxidase catalyzes the reaction of a lower primary alcohol or an unsaturated alcohol and oxygen to produce the corresponding aldehyde and hydrogen peroxide. Amine oxidase catalyzes the reaction of an amine, typically a primary amine, but also, in some cases, a secondary or tertiary amine , water, and oxygen to produce the corresponding aldehyde, ammonia, and hydrogen peroxide. In an illustrative reaction, glucose oxidase catalyzes the reaction of β-D-glucose, water, and oxygen during application to the outer ear to produce hydrogen peroxide and gluconic acid.

The properties of a number of preferred oxidases suitable for use in compositions according to the present invention are known. For example, glucose oxidase from *Aspergillus niger* has been determined to have a molecular weight of 150,000 (Pazur et al. (1965)). The enzyme is a glycoprotein containing two molecules of the redox coenzyme flavin adenine dinucleotide (FAD). The amino acid composition has been determined. The isoelectric point of the enzyme is 4.2. The optimum pH of the enzyme is 5.5 with a broad pH range of from 4 to 7. Inhibitors of the enzyme include monovalent silver ions and divalent mercury and copper ions.

Galactose oxidase from *Dactylium dendroides* has a molecular weight of 42,000. It is a metalloenzyme containing one gram-atom of copper per mole. The amino acid composition has been determined. The optimum pH of the enzyme is 7.

Urate oxidase (uricase) from hog liver or beef liver has a molecular weight of 100,000. It is a metalloenzyme containing one gram-atom of copper per mole. The isoelectric point of the enzyme is 6.3. The optimum pH of the enzyme is 9.

D-amino acid oxidase from hog kidney has a molecular weight of 90,000. The enzyme is a glycoprotein containing two molecules of flavin adenine dinucleotide. The optimum pH of the enzyme is 9.1. Certain heavy metals are inhibitors of the enzyme.

The oxidizable substrate is typically present in the composition at a concentration of from about 0.015 millimoles per milliliter of liquid to about 0.6 millimoles per gram of composition. Preferably, the oxidizable substrate is present in the composition at a concentration of from about 0.025 millimoles per gram of composition to about 0.1 millimole per gram of composition. The salt that acts as an oxygen acceptor is typically present in the composition at a concentration of from about 0.0001 millimole to about 0.01 millimole per gram of composition. The salt that acts as an oxygen acceptor is preferably present in the composition at a concentration of from about 0.001 millimole to about 0.006 millimole per gram of composition.

Typically, the oxidoreductase enzyme is present in the composition in a concentration of from about 0.5 IU to about 500 IU per gram of composition. Preferably, the oxidoreductase enzyme is present in the composition in a concentration of from about 10 IU to about 40 IU per gram of composition. Oxidoreductase enzymes are supplied in dry or liquid form with the label specifying the concentration in International Units on a per gram or per milliliter basis, as appropriate.

A particularly preferred oxidase is glucose oxidase. If glucose oxidase is included in a composition according to the present invention, a preferred substrate for the glucose oxidase, to be included in the composition, is β-D-glucose. If another oxidase enzyme is used, appropriate substrates are described above.

In particular, the following combinations of glycoside linkage-hydrolyzing enzymes and peroxidases, if present, can be used in compositions according to the present invention: (1) pectinase as the glycoside linkage-hydrolyzing enzyme; (2) dextranase and pectinase as the glycoside linkage-hydrolyzing enzymes; (3) dextranase and pectinase as the glycoside linkage-hydrolyzing enzymes, plus lactoperoxidase as the peroxidase; (4) pectinase as the glycoside linkage-hydrolyzing enzyme, plus lactoperoxidase as the peroxidase; (5) dextranase and xylanase as the glycoside linkage-hydrolyzing enzymes; (6) α-galactosidase and amylase as the glycoside linkage-hydrolyzing enzymes; (7) pectinase and amylase as the glycoside linkage-hydrolyzing enzymes, plus lactoperoxidase as the peroxidase; (8) dextranase, pectinase, and β-D-glucosidase as the glycoside linkage-hydrolyzing enzymes, plus lactoperoxidase as the peroxidase; (9) dextranase, pectinase, and cellulase as the glycoside linkage-hydrolyzing enzymes, plus lactoperoxidase as the peroxidase; and (10) dextranase, pectinase, cellulase, amylase, and xylanase as the glycoside linkage-hydrolyzing enzymes, plus lactoperoxidase as the peroxidase. Other combinations are possible. These combinations can be combined with lysozyme and/or lactoferrin. Additionally, as indicated above, glucose oxidase or another oxidase can be included as a source of peroxide, plus a substrate for the oxidase such as β-D-glucose.

Other ingredients generally known in the pharmaceutical art can be incorporated into compositions according to the present invention, including colorants, chelating agents, preservatives, and stabilizers, with the proviso that these additional ingredients do not inhibit the hydrolytic and oxidation-reduction reactions on which the activity of the compositions according to the present invention depend.

The composition can further comprise a thickener to provide the composition with an enzyme immobilizing viscosity which inhibits enzymatic action during processing and in packing. A preferred thickener is hydroxypropylcellulose (Klucel). Other thickeners are known in the art and can be alternatively used. These thickeners include hydroxymethyl cellulose, methyl cellulose, polyvinylpyrrolidone (PVP), PVM, PVM/MA copolymers, xanthan gum, and mixtures thereof.

The composition can be aqueous or non-aqueous. If the composition is aqueous, the concentration of water (w/w) typically is from about 0.150% to about 5.441%. However, the composition can be a non-aqueous composition with substantially no water content.

In one alternative, the composition includes from about 35% to about 75% of a hydrocarbon. Typically the hydrocarbon is an isoprenoid or a derivative of an isoprenoid. If the hydrocarbon is an isoprenoid or a derivative of an isoprenoid, preferably the hydrocarbon has from four to six isoprene units. More preferably, the hydrocarbon has six isoprene units. A particularly suitable hydrocarbon is squalene. Other hydrocarbons can be used.

In one preferred alternative, the composition is formulated to treat otitis media. As used herein, the terms "treat," "treating," "treatment," and analogous terminology does not imply a cure for otitis media or any other disease or condition; rather, this terminology is used to refer to any clinically detectable improvement in the disease or condition being treated, including, but not limited to, reduction in bacterial numbers or viability, reduction in fever, reduction in pain, reduction in hearing loss, reduction in fluid effusion, improvement in subjective well-being experienced by the patient, or any other clinically detectable improvement.

In another preferred alternative, the composition is formulated to treat otitis externa.

In another preferred alternative, the composition is formulated to treat infection by *Pseudomonas aeruginosa.*

The physical form of a composition according to the present invention can be, for example, a solution, a gel, a cream, or a solid, depending on the exact composition and the method of administration chosen, as well as the site of administration and whether the composition is intended to treat otitis media or otitis externa. If the solution is a gel, the viscosity of the gel can be chosen to provide efficient application by the user according to general principles of gel composition for pharmaceutical compositions. The particular gel former or gel formers used in a particular composition and their concentrations can be determined by one of ordinary skill in the art.

Compositions according to the present invention can include additional components, such as, but not limited to, a gel forming component, a lipophilic component, a wax, a skin soothing component, an emulsifier component, a bulk adding component, a gum component, or other components such as are generally used in pharmaceutical compositions intended for application to the ear canal, such as stabilizers, buffers, a colorant, a fragrance, or a preservative. In particular, compositions according to the present invention can include one or more of the following components: (1) benzyl alcohol; (2) glycerol; (3) dipropylene glycol; (4) tripropylene glycol; (5) xanthan gum; (6) PEG-20 almond glyceride; (7) an isopropyl ester of a long chain fatty acid selected from the group consisting of isopropyl myristate, isopropyl laurate, and isopropyl stearate, preferably isopropyl myristate; (8) aloe vera; (9) sodium polyacrylate/polyacrylic acid; (10) beeswax; (11) PEG-40 stearate; (12) polyethylene glycol; and (13) Polawax.

Compositions according to the present invention can be formulated by techniques known in the art, including techniques that are conventional in the cosmetic art and in the art of over-the-counter and prescription drug composition for blending lipid-soluble components and water-soluble components for the preparation of liquids, gels, creams, or suppositories. These mixing techniques include both manual and mechanical mixing, and include homogenization mixing and sweep mixing. The mixing techniques to be used can be chosen by one of ordinary skill in the art based on variables such as the viscosity of the components to be mixed and the volume of those components, as well as the relative proportion of lipid-soluble and water-soluble ingredients, the proportion of water, and the final physical form of the desired composition.

Particular embodiments of compositions according to the present invention, include, but are not limited to the following:

Formulation 1 is an aqueous composition including the enzyme pectinase. In these formulations, percentages are given in terms of (w/w).

Typically, Formulation 1 comprises:
(1) from about 12.33% to about 18.49% of glycerol;
(2) from about 71.661% to about 83.986% of propylene glycol;
(3) from about 0.352% to about 0.528% of hydroxypropylcellulose;
(4) from about 2.405% to about 3.607% of benzyl alcohol;
(5) from about 0.0072% to about 0.0108% of pectinase; and
(6) from about 0.92% to about 1.38% of water.

Preferably, Formulation 1 comprises:
(1) about 15.41% of glycerol;
(2) about 79.623% of propylene glycol;
(3) about 0.440% of hydroxypropylcellulose;
(4) about 3.006% of benzyl alcohol;
(5) about 0.009% of pectinase; and
(6) about 1.150% of water.

Formulation 2 is an aqueous composition including the enzyme pectinase and hydrocortisone as an anti-inflammatory agent.

Typically, Formulation 2 comprises:
(1) from about 28.328% to about 42.492% of glycerol;
(2) from about 52.761% to about 64.485% of propylene glycol;
(3) from about 1.152% to about 1.728% of tripropylene glycol;
(4) from about 2.405% to about 3.607% of benzyl alcohol;
(5) from about 0.80% to about 1.20% of hydrocortisone;
(6) from about 0.008% to about 0.012% of pectinase; and
(7) from about 0.80% to about 1.20% of water.

Preferably, Formulation 2 comprises:
(1) about 35.410% of glycerol;
(2) about 58.623% of propylene glycol;
(3) about 1.440% of tripropylene glycol;
(4) about 3.006% of benzyl alcohol;
(5) about 1.00% of hydrocortisone;
(6) about 0.010% of pectinase; and
(7) about 1.00% of water.

Formulation 3 is an aqueous composition including the two enzymes dextranase and pectinase.

Typically, Formulation 3 comprises:
(1) from about 24.328% to about 36.492% of glycerol;
(2) from about 57.261 % to about 69.985% of propylene glycol;
(3) from about 1.152% to about 1.728% of tripropylene glycol;
(4) from about 2.405% to about 3.607% of benzyl alcohol;
(5) from about 0.008% to about 0.012% of dextranase;
(6) from about 0.008% to about 0.012% of pectinase; and
(7) from about 1.201 % to about 1.801 % of water.

Preferably, Formulation 3 comprises:
(1) about 30.410% of glycerol;
(2) about 63.623% propylene glycol;
(3) about 1.440% of tripropylene glycol;
(4) about 3.006% of benzyl alcohol;
(5) about 0.010% of dextranase;
(6) about 0.010% of pectinase; and
(7) about 1.501 % of water.

Formulation 4 is an aqueous composition including dextranase with hydroxypropylcellulose as a thickener.

Typically, Formulation 4 comprises:
(1) from about 16.328% to about 24.492% of glycerol;
(2) from about 63.561 % to about 76.506% of propylene glycol;
(3) from about 0.40% to about 0.60% of hydroxypropylcellulose;
(4) from about 2.405% to about 3.607% of benzyl alcohol;
(5) from about 0.008% to about 0.012% of dextranase; and
(6) from about 4.353% to about 6.529% of water.

Preferably, Formulation 4 comprises:
(1) about 20.410% of glycerol;
(2) about 70.623% of propylene glycol;
(3) about 0.50% of hydroxypropylcellulose;
(4) about 3.006% of benzyl alcohol;
(5) about 0.010% of dextranase; and
(6) about 5.441 % of water.

Formulation 5 is an aqueous composition including dextranase and pectinase, and further including lactoperoxidase as a source of hydrogen peroxide. Formulation 5 further includes hydroxypropylcellulose.

Typically, Formulation 5 comprises:
(1) from about 16.328% to about 24.492% of glycerol;
(2) from about 63.561 % to about 76.490% of propylene glycol;
(3) from about 0.40% to about 0.60% of hydroxypropylcellulose;
(4) from about 2.405% to about 3.607% of benzyl alcohol;
(5) from about 0.008% to about 0.012% of dextranase;
(6) from about 0.008% to about 0.012% of lactoperoxidase;
(7) from about 0.008% to about 0.012% of pectinase; and
(8) from about 4.353% to about 6.529% of water.

Preferably, Formulation 5 comprises:
(1) about 20.410% of glycerol;
(2) about 70.623% of propylene glycol;
(3) about 0.50% of hydroxypropylcellulose;
(4) about 3.006% of benzyl alcohol;
(5) about 0.010% of dextranase;
(6) about 0.010% of lactoperoxidase;
(7) about 0.010% of pectinase; and
(8) about 5.441 % of water.

Formulation 6 is an aqueous composition including dextranase and pectinase, and further including lysozyme and lactoferrin. Formulation 6 further includes hydroxypropylcellulose.

Typically, Formulation 6 comprises:
(1) from about 20.328% to about 30.492% of glycerol;
(2) from about 59.061% to about 72.185% of propylene glycol;
(3) from about 0.40% to about 0.60% of hydroxypropylcellulose;
(4) from about 2.405% to about 3.607% of benzyl alcohol;
(5) from about 0.008% to about 0.012% of lysozyme;
(6) from about 0.008% to about 0.012% of lactoferrin;
(7) from about 0.008% to about 0.012% of lactoperoxidase;
(8) from about 0.008% to about 0.012% of pectinase; and
(9) from about 4.353% to about 6.529% of water.

Preferably, Formulation 6 comprises:
(1) about 25.410% of glycerol;
(2) about 65.523% of propylene glycol;
(3) about 0.50% of hydroxypropylcellulose;
(4) about 3.006% of benzyl alcohol;
(5) about 0.010% of lysozyme;
(6) about 0.010% of lactoferrin;
(7) about 0.010% of lactoperoxidase;
(8) about 0.010% of pectinase; and
(9) about 5.441 % of water.

Formulation 7 is an aqueous composition including dextranase, lactoperoxidase, and pectinase. Formulation 7 omits benzyl alcohol.

Typically, Formulation 7 comprises:
(1) from about 16.830% to about 25.246% of glycerol;
(2) from about 65.518% to about 78.248% of propylene glycol;
(3) from about 0.412% to about 0.618% of hydroxypropylcellulose;
(4) from about 0.00824% to about 0.0124% of dextranase;
(5) from about 0.00824% to about 0.0124% of lactoperoxidase;
(6) from about 0.00824% to about 0.0124% of pectinase; and
(7) from about 4.486% to about 6.730% of water.

Preferably, Formulation 7 comprises:
(1) about 21.038% of glycerol;
(2) about 72.798% of propylene glycol;
(3) about 0.515% of hydroxypropylcellulose;
(4) about 0.0103% of dextranase;
(5) about 0.0103% of lactoperoxidase;
(6) about 0.0103% of pectinase; and
(7) about 5.608% of water.

Formulation 8 is an aqueous composition including dextranase, lactoperoxidase, and pectinase and that includes glycerol, propylene glycol, and tripropylene glycol. Formulation 8 omits benzyl alcohol.

Typically, Formulation 8 comprises:
(1) from about 25.201% to about 37.813% of glycerol;
(2) from about 47.114% to about 57.584% of propylene glycol;
(3) from about 8.375% to about 12,563% of tripropylene glycol;
(4) from about 0.00832% to about 0.0125% of dextranase;
(5) from about 0.00832% to about 0.0125% of lactoperoxidase;
(6) from about 0.00832% to about 0.0125% of pectinase; and
(7) from about 4.510% to about 6.766% of water.

Preferably, Formulation 8 comprises:
(1) about 31.511% of glycerol;
(2) about 52.349% of propylene glycol;
(3) about 10.469% of tripropylene glycol;
(4) about 0.0104% of dextranase;
(5) about 0.0104% of lactoperoxidase;
(6) about 0.0104% of pectinase; and
(7) about 5.638% of water.

Formulation 9 is an aqueous composition including dextranase and xylanase and that includes glycerol, propylene glycol, and tripropylene glycol. Formulation 9 omits benzyl alcohol.

Typically, Formulation 9 comprises:
(1) from about 16.919% to about 25.379% of glycerol;
(2) from about 56.440% to about 68.982% of propylene glycol;
(3) from about 8.383% to about 12.575% of tripropylene glycol;
(4) from about 0.00832% to about 0.0125% of dextranase;
(5) from 0.00832% to about 0.0125% of xylanase; and
(6) from about 4.510% to about 6.766% of water.

Preferably, Formulation 9 comprises:
(1) about 21.149% of glycerol;
(2) about 62.711 % of propylene glycol;
(3) about 10.479% of tripropylene glycol;
(4) about 0.104% of dextranase;
(5) about 0.104% of xylanase; and
(6) about 5.638% of water.

Formulation 10 is an aqueous composition including α-galactosidase and amylase and that includes glycerol and propylene glycol. Formulation 10 omits benzyl alcohol.

Typically, Formulation 10 comprises:
(1) from about 12.348% to about 18.522% of glycerol;
(2) from about 73.051 % to about 84.927% of propylene glycol;
(3) from about 0.460% to about 0.690% of hydroxypropylcellulose;
(4) from about 0.0092% to about 0.0138% of α-galactosidase;
(5) from about 0.0092% to about 0.0138% of amylase; and
(6) from about 2.247% to about 3.371 % of water.

Preferably, Formulation 10 comprises:
(1) about 15.435% of glycerol;
(2) about 81.168% of propylene glycol;
(3) about 0.575% of hydroxypropylcellulose;
(4) about 0.0115% of α-galactosidase;
(5) about 0.0115% of amylase; and
(6) about 2.809% of water.

Formulation 11 is an aqueous composition including lactoperoxidase, pectinase, and amylase and that includes glycerol and propylene glycol. Formulation 11 omits benzyl alcohol.

Typically, Formulation 11 comprises:
(1) from about 14.596% to about 21.894% of glycerol;
(2) from about 69.818% to about 82.069% of propylene glycol;
(3) from about 0.00944% to about 0.0141% of lactoperoxidase;
(4) from about 0.0474% to about 0.0710% of pectinase;
(5) from about 0.0190% to about 0.0284% of amylase; and
(6) from about 3.259% to about 4.889% of water.

Preferably, Formulation 11 comprises:
(1) about 18.245% of glycerol;
(2) about 77.576% of propylene glycol;
(3) about 0.0118% of lactoperoxidase;
(4) about 0.0592% of pectinase;
(5) about 0.0237% of amylase; and
(6) about 4.074% of water.

Formulation 12 is an aqueous composition including dextranase, lactoperoxidase, and pectinase. Formulation 12 includes glycerol, propylene glycol, and tripropylene glycol. Formulation 12 further includes potassium thiocyanate. Formulation 12 omits benzyl alcohol.

Typically, Formulation 12 comprises:
(1) from about 25.204% to about 37.806% of glycerol;
(2) from about 47.105% to about 57.572% of propylene glycol;
(3) from about 8.374% to about 12.560% of tripropylene glycol;
(4) from about 0.00832% to about 0.0125% of dextranase;
(5) from about 0.0166% to about 0.0248% of lactoperoxidase;
(6) from about 0.00832% to about 0.0125% of pectinase;
(7) from about 0.00832% to about 0.0125% of potassium thiocyanate;
   and
(8) from about 4.510% to about 6.764% of water.

Preferably, Formulation 12 comprises:
(1) about 31.505% of glycerol;
(2) about 52.339% of propylene glycol;
(3) about 10.467% of tripropylene glycol;
(4) about 0.0104% of dextranase;
(5) about 0.0207% of lactoperoxidase;
(6) about 0.0104% of pectinase;
(7) about 0.0104% of potassium thiocyanate; and
(8) about 5.637% of water.

Formulation 13 is a non-aqueous composition including dextranase and lactoperoxidase. Formulation 13 includes glycerol and propylene glycol. Formulation 13 further includes potassium thiocyanate. Formulation 13 omits benzyl alcohol.

Typically, Formulation 13 comprises:
(1) from about 12.846% to about 19.268% of glycerol;
(2) from about 75.510% to about 87.112% of propylene glycol;
(3) from about 0.00832% to about 0.0125% of dextranase;
(4) from about 0.0166% to about 0.0250% of lactoperoxidase;
(5) from about 0.00832% to about 0.0125% of pectinase; and
(6) from about 0.00832% to about 0.0125% of potassium thiocyanate.

Preferably, Formulation 13 comprises:
(1) about 16.057% of glycerol;
(2) about 83.901 % of propylene glycol;
(3) about 0.0104% of dextranase;
(4) about 0.0208% of lactoperoxidase;
(5) about 0.0104% of pectinase; and
(6) about 0.0104% of potassium thiocyanate.

Formulation 14 is a non-aqueous formulation including dextranase, lactoperoxidase, and pectinase. Formulation 14 also includes lysozyme and potassium iodate. Formulation 14 includes glycerol and propylene glycol; it omits benzyl alcohol.

Typically, Formulation 14 comprises:
(1) from about 12.834% to about 19.250% of glycerol;
(2) from about 75.439% to about 87.100% of propylene glycol;
(3) from about 0.00832% to about 0.0125% of dextranase;
(4) from about 0.0166% to about 0.0250% of lactoperoxidase;
(5) from about 0.0166% to about 0.0250% of pectinase;
(6) from about 0.0166% to about 0.0250% of lysozyme; and
(7) from about 0.00832% to about 0.0125% of potassium iodate.

Preferably, Formulation 14 comprises:
(1) about 16.042% of glycerol;
(2) about 83.821% of propylene glycol;
(3) about 0.0104% of dextranase;
(4) about 0.0208% of lactoperoxidase;
(5) about 0.0208% of pectinase;
(6) about 0.0208% of lysozyme; and
(7) about 0.0104% of potassium iodate.

Formulation 15 is a non-aqueous composition including dextranase, lactoperoxidase, and pectinase. Formulation 15 includes glycerol, propylene glycol, and tripropylene glycol, as well as lysozyme, lactoferrin, and potassium iodate. Formulation 15 omits benzyl alcohol.

Typically, Formulation 15 comprises:
(1) from about 8.670% to about 13.004% of glycerol;
(2) from about 75.439% to about 87.091 % of propylene glycol;
(3) from about 4.164% to about 6.246% of tripropylene glycol;
(4) from about 0.00832% to about 0.0125% of dextranase;
(5) from about 0.0166% to about 0.0250% of lactoperoxidase;
(6) from about 0.0166% to about 0.0250% of pectinase;
(7) from about 0.0166% to about 0.0250% of lysozyme;
(8) from about 0.00832% to about 0.0125% of lactoferrin; and
(9) from about 0.00832% to about 0.0125% of potassium iodate.

Preferably, Formulation 15 comprises:
(1) about 10.847% of glycerol;
(2) about 83.821% of propylene glycol;
(3) about 5.205% of tripropylene glycol;
(4) about 0.0104% of dextranase;
(5) about 0.0208% of lactoperoxidase;
(6) about 0.0208% of pectinase;
(7) about 0.0208% of lysozyme;
(8) about 0.0104% of lactoferrin; and
(9) about 0.0104% of potassium iodate.

Formulation 16 is a non-aqueous composition including dextranase, lactoperoxidase, pectinase, and β-D-glucosidase. Formulation 16 further includes potassium iodate, as well as glycerol, propylene glycol, and tripropylene glycol. Formulation 16 omits benzyl alcohol.

Typically, Formulation 16 comprises:
(1) from about 8.678% to about 13.016% of glycerol;
(2) from about 75.512% to about 87.104% of propylene glycol;
(3) from about 4.168% to about 6.252% of tripropylene glycol;
(4) from about 0.00832% to about 0.0125% of dextranase;
(5) from about 0.0166% to about 0.0250% of lactoperoxidase;
(6) from about 0.00832% to about 0.0125% of pectinase;
(7) from about 0.00832% to about 0.0125% of β-D-glucosidase;
(8) from about 0.00832% to about 0.0125% of potassium iodate.

Preferably, Formulation 16 comprises:
(1) about 10.847% of glycerol;
(2) about 83.902% of propylene glycol;
(3) about 5.210% of tripropylene glycol;
(4) about 0.0104% of dextranase;
(5) about 0.0208% of lactoperoxidase;
(6) about 0.0104% of pectinase;
(7) about 0.0104% of β-D-glucosidase; and
(8) about 0.0104% of potassium iodate.

Formulation 17 is a non-aqueous formulation that includes dextranase, lactoperoxidase, pectinase, and cellulase. Formulation 17 includes glycerol, propylene glycol, and tripropylene glycol. Formulation 17 further includes potassium thiocyanate. Formulation 17 omits benzyl alcohol.

Typically, Formulation 17 comprises:
(1) from about 17.014% to about 25.520% of glycerol;
(2) from about 66.134% to about 78.768% of propylene glycol;
(3) from about 4.168% to about 6.252% of tripropylene glycol;
(4) from about 0.00832% to about 0.0125% of dextranase;
(5) from about 0.0166% to about 0.0250% of lactoperoxidase;
(6) from about 0.00832% to about 0.0125% of pectinase;
(7) from about 0.00832% to about 0.0125% of cellulase; and
(8) from about 0.00832% to about 0.0125% of potassium thiocyanate.

Preferably, Formulation 17 comprises:
(1) about 21.267% of glycerol;
(2) about 73.482% of propylene glycol;
(3) about 5.210% of tripropylene glycol;
(4) about 0.0104% of dextranase;
(5) about 0.0208% of lactoperoxidase;
(6) about 0.0104% of pectinase;
(7) about 0.0104% of cellulase; and
(8) about 0.0104% of potassium thiocyanate.

Formulation 18 is a non-aqueous composition including dextranase, lactoperoxidase, pectinase, cellulase, amylase, and xylanase, as well as potassium thiocyanate. Formulation 18 includes glycerol, propylene glycol, and tripropylene glycol. Formulation 18 omits benzyl alcohol.

Typically, Formulation 18 comprises:
(1) from about 16.998% to about 25.496% of glycerol;
(2) from about 66.070% to about 78.763% of propylene glycol;
(3) from about 4.164% to about 6.246% of tripropylene glycol;
(4) from about 0.00832% to about 0.0125% of dextranase;
(5) from about 0.0166% to about 0.0250% of lactoperoxidase;
(6) from about 0.00832% to about 0.0125% of pectinase;
(7) from about 0.00832% to about 0.0125% of cellulase;
(8) from about 0.0166% to about 0.0250% of amylase;
(9) from about 0.00832% to about 0.0125% of xylanase; and
(10) from about 0.00832% to about 0.0125% of potassium thiocyanate.

Preferably, Formulation 18 comprises:
(1) about 21.247% of glycerol;
(2) about 73.411 % of propylene glycol;
(3) about 5.205% of tripropylene glycol;
(4) about 0.0104% of dextranase;
(5) about 0.0208% of lactoperoxidase;
(6) about 0.0104% of pectinase;
(7) about 0.0104% of cellulase;
(8) about 0.0208% of amylase;
(9) about 0.0104% of xylanase; and
(10) about 0.0104% of potassium thiocyanate.

Formulation 19 is a non-aqueous composition including dextranase, lactoperoxidase, glucose oxidase, pectinase, cellulase, amylase, and xylanase, as well as potassium iodate. Formulation 19 includes glycerol and propylene glycol. Formulation 19 also includes β-D-glucose. Formulation 19 includes benzyl alcohol.

Typically, Formulation 19 comprises:
(1) from about 19.311% to about 28.967% of glycerol;
(2) from about 65.497% to about 78.212% of propylene glycol;
(3) from about 2.194% to about 3.296% of benzyl alcohol;
(4) from about 0.220% to about 0.330% of β-D-glucose;
(5) from about 0.00731% to about 0.0110% of dextranase;
(6) from about 0.00658% to about 0.00988% of lactoperoxidase;
(7) from about 0.00585% to about 0.00877% of glucose oxidase;
(8) from about 0.00658% to about 0.00988% of pectinase;
(9) from about 0.00731% to about 0.0110% of dextranase;
(10) from about 0.0146% to about 0.0220% of amylase;
(11) from about 0.00731% to about 0.0110% of xylanase; and
(12) from about 0.00731 % to about 0.0110% of potassium iodate.

Preferably, Formulation 19 comprises:
(1) about 24.139% of glycerol;
(2) about 72.775% of propylene glycol;
(3) about 2.747% of benzyl alcohol;
(4) about 0.275% of β-D-glucose;
(5) about 0.00914% of dextranase;
(6) about 0.00823% of lactoperoxidase;
(7) about 0.00731% of glucose oxidase;
(8) about 0.00823% of pectinase;
(9) about 0.00914% of cellulase;
(10) about 0.0183% of amylase;
(11) about 0.00914% of xylanase; and
(12) about 0.00914% of potassium iodate.

Formulation 20 is a non-aqueous composition including dextranase, lactoperoxidase, glucose oxidase, and pectinase. Formulation 20 includes glycerol and propylene glycol. Formulation 20 further includes hydrocortisone and potassium iodate. Formulation 20 also further includes benzyl alcohol.

Typically, Formulation 20 comprises:
(1) from about 19.142% to about 28.712% of glycerol;
(2) from about 64.924% to about 77.702% of propylene glycol;
(3) from about 2.178% to about 3.268% of benzyl alcohol;
(4) from about 0.725% to about 1.087% of hydrocortisone;
(5) from about 0.218% to about 0.328% of β-D-glucose;
(6) from about 0.00725% to about 0.0109% of dextranase;
(7) from about 0.00652% to about 0.00978% of lactoperoxidase;
(8) from about 0.00580% to about 0.00870% of glucose oxidase;
(9) from about 0.00725% to about 0.0109% of pectinase; and
(10) from about 0.00725% to about 0.0109% of potassium iodate.

Preferably, Formulation 20 comprises:
(1) about 23.927% of glycerol;
(2) about 72.138% of propylene glycol;
(3) about 2.723% of benzyl alcohol;
(4) about 0.906% of hydrocortisone;
(5) about 0.273% of β-D-glucose;
(6) about 0.00906% of dextranase;
(7) about 0.00815% of lactoperoxidase;
(8) about 0.00725% of glucose oxidase;
(9) about 0.00906% of pectinase; and
(10) about 0.00906% of potassium iodate.

Formulation 21 is an aqueous composition containing a minimal amount of water. Formulation 21 includes lactoperoxidase, glucose oxidase, and pectinase, and β-D-glucose. Formulation 21 includes glycerol and propylene glycol, as well as hydroxypropylcellulose. Formulation 21 further includes hydrocortisone and benzyl alcohol, as well as lactoferrin and lysozyme.

Typically, Formulation 21 comprises:
(1) from about 28.328% to about 42.492% of glycerol;
(2) from about 52.761 % to about 64.485% of propylene glycol;
(3) from about 1.152% to about 1.728% of hydroxypropylcellulose;
(4) from about 2.405% to about 3.607% of benzyl alcohol;
(5) from about 0.120% to about 0.180% of water;
(6) from about 0.800% to about 1.200% of hydrocortisone;
(7) from about 0.241 % to about 0.361 % of β-D-glucose;
(8) from about 0.0064% to about 0.0096% of lactoperoxidase;
(9) from about 0.0008% to about 0.0012% of glucose oxidase;
(10) from about 0.0064% to about 0.0096% of lactoferrin;
(11) from about 0.0064% to about 0.0096% of lysozyme;
(12) from about 0.0080% to about 0.0120% of pectinase; and
(13) from about 0.028% to about 0.042% of potassium iodate.

Preferably, Formulation 21 comprises:
(1) about 35.410% of glycerol;
(2) about 58.623% of propylene glycol;
(3) about 1.440% of hydroxypropylcellulose;
(4) about 3.006% of benzyl alcohol;
(5) about 0.150% of water;
(6) about 1.000% of hydrocortisone;
(7) about 0.301% of β-D-glucose;
(8) about 0.008% of lactoperoxidase;
(9) about 0.001 % of glucose oxidase;
(10) about 0.008% of lactoferrin;
(11) about 0.008% of lysozyme;
(12) about 0.010% of pectinase; and
(13) about 0.035% of potassium iodate.

Formulation 22 is the same as Formulation 21 except that it substitutes potassium thiocyanate in Formulation 22, for potassium iodate in Formulation 21.

Typically, Formulation 22 comprises:
(1) from about 28.328% to about 42.492% of glycerol;
(2) from about 52.761% to about 64.485% of propylene glycol;
(3) from about 1.152% to about 1.728% of hydroxypropylcellulose;
(4) from about 2.405% to about 3.607% of benzyl alcohol;
(5) from about 0.120% to about 0.180% of water;
(6) from about 0.800% to about 1.200% of hydrocortisone;
(7) from about 0.241 % to about 0.361 % of β-D-glucose;
(8) from about 0.0064% to about 0.0096% of lactoperoxidase;
(9) from about 0.0008% to about 0.0012% of glucose oxidase;
(10) from about 0.0064% to about 0.0096% of lactoferrin;
(11) from about 0.0064% to about 0.0096% of lysozyme;
(12) from about 0.0080% to about 0.0120% of pectinase; and
(13) from about 0.028% to about 0.042% of potassium thiocyanate.

Preferably, Formulation 22 comprises:
(1) about 35.410% of glycerol;
(2) about 58.623% of propylene glycol;
(3) about 1.440% of hydroxypropylcellulose;
(4) about 3.006% of benzyl alcohol;
(5) about 0.150% of water;
(6) about 1.000% of hydrocortisone;
(7) about 0.301% of β-D-glucose;
(8) about 0.008% of lactoperoxidase;
(9) about 0.001 % of glucose oxidase;
(10) about 0.008% of lactoferrin;
(11) about 0.008% of lysozyme;
(12) about 0.010% of pectinase; and
(13) about 0.035% of potassium thiocyanate.

Formulation 23 is an aqueous composition with a minimal amount of water that includes lactoperoxidase, glucose oxidase, pectinase, and dextranase. Formulation 23 includes glycerol and propylene glycol, as well as hydroxypropylcellulose. Formulation 23 further includes lactoferrin and lysozyme, as well as β-D-glucose and potassium thiocyanate. Formulation 23 further includes benzyl alcohol.

Typically, Formulation 23 comprises:
(1) from about 28.328% to about 42.492% of glycerol;
(2) from about 53.652% to about 65.574% of propylene glycol;
(3) from about 1.152% to about 1.728% of hydroxypropylcellulose;
(4) from about 2.405% to about 3.607% of benzyl alcohol;
(5) from about 0.120% to about 0.180% of water;
(6) from about 0.241 % to about 0.361 % of β-D-glucose;
(7) from about 0.0064% to about 0.0096% of lactoperoxidase;
(8) from about 0.0008% to about 0.0012% of glucose oxidase;
(9) from about 0.0064% to about 0.0096% of lactoferrin;
(10) from about 0.0064% to about 0.0096% of lysozyme;
(11) from about 0.0080% to about 0.0120% of pectinase;
(12) from about 0.0080% to about 0.0120% of dextranase; and
(13) from about 0.028% to about 0.042% of potassium thiocyanate.

Preferably, Formulation 23 comprises:
(1) about 35.410% of glycerol;
(2) about 59.613% of propylene glycol;
(3) about 1.440% of hydroxypropylcellulose;
(4) about 3.006% of benzyl alcohol;
(5) about 0.150% of water;
(6) about 0.301 % of β-D-glucose;
(7) about 0.008% of lactoperoxidase;
(8) about 0.001 % of glucose oxidase;
(9) about 0.008% of lactoferrin;
(10) about 0.008% of lysozyme;
(11) about 0.010% of pectinase;
(12) about 0.010% of dextranase;
(13) about 0.035% of potassium thiocyanate.

Formulation 24 is an aqueous composition that also includes the isoprenoid hydrocarbon squalene and that includes lactoperoxidase and pectinase. Formulation 24 includes propylene glycol and glycerol. Formulation 24 also includes lactoferrin and lysozyme, as well as potassium thiocyanate as a substrate for the lactoperoxidase. Formulation 24 omits benzyl alcohol.

Typically, Formulation 24 comprises:
(1) from about 61.2% to about 74.4% of squalene;
(2) from about 6.8% to about 10.2% of propylene glycol;
(3) from about 16.32% to about 24.48% of glycerol;
(4) from about 2.4% to about 3.6% of water;
(5) from about 0.012% to about 0.018% of lactoperoxidase;
(6) from about 0.008% to about 0.012% of lactoferrin;
(7) from about 0.008% to about 0.012% of lysozyme;
(8) from about 0.048% to about 0.072% of potassium thiocyanate; and
(9) from about 0.0096% to about 0.0144% of pectinase.

Preferably, Formulation 24 comprises:
(1) about 68% of squalene;
(2) about 8.5% of propylene glycol;
(3) about 20.4% of glycerol;
(4) about 3% of water;
(5) about 0.015% of lactoperoxidase;
(6) about 0.010% of lactoferrin;
(7) about 0.010% of lysozyme;
(8) about 0.060% of potassium thiocyanate; and
(9) about 0.012% of pectinase.

Formulation 25 is an aqueous composition that also includes the isoprenoid hydrocarbon squalene and that includes lactoperoxidase and amylase. Formulation 25 includes propylene glycol and glycerol. Formulation 25 also includes lactoferrin and lysozyme, as well as potassium iodate as a substrate for the lactoperoxidase. Formulation 25 omits benzyl alcohol.

Typically, Formulation 25 comprises:
(1) from about 38.16% to about 46.64% of squalene;
(2) from about 5.2% to about 7.8% of propylene glycol;
(3) from about 43.2% to about 52;8% of glycerol;
(4) from about 2.4% to about 3.6% of water;
(5) from about 0.012% to about 0.018% of lactoperoxidase;
(6) from about 0.008% to about 0.012% of lactoferrin;
(7) from about 0.008% to about 0.012% of lysozyme;
(8) from about 0.032% to about 0.048% of potassium iodate; and
(9) from about 0.0096% to about 0.0144% of amylase.

Preferably, Formulation 25 comprises:
(1) about 42.4% of squalene;
(2) about 6.5% of propylene glycol;
(3) about 48% of glycerol;
(4) about 3% of water;
(5) about 0.015% of lactoperoxidase;
(6) about 0.010% of lactoferrin;
(7) about 0.010% of lysozyme;
(8) about 0.04% of potassium iodate; and
(9) about 0.012% of amylase.

In another alternative, a composition according to the present invention can further include an antibiotic that is effective in the treatment of *P. aeruginosa* in a quantity effective to exert a bactericidal action against *P. aeruginosa.* These antibiotics are described above.

Other formulations can be prepared that are similar to the ones described in detail above.

Another embodiment of the present invention is a method of treating an ear infection comprising the step of administering a quantity of a composition according to the present invention as described above to a subject with an ear infection in order to treat the infection. The precise therapeutically effective amount for a subject will depend upon the subject's age, size, weight, and health, the extent of the ear infection, the bacterium causing the ear infection, the presence of other conditions such as allergic reactions that can complicate the ear infection, and the therapeutics or combination of therapeutics selected for administration, as well as variables such as liver and kidney function that affect the pharmacokinetics of administered therapeutics. Thus, it is not useful to specify an exact effective amount in advance. However, the effective amount for a given situation can be determined by routine experimentation and is within the judgment of the clinician. The frequency of administration, as well, can be determined by one of ordinary skill in the art with reference to the above parameters.

Typically, the ear infection is otitis externa or otitis media. Typically, the ear infection is caused by *Pseudomonas aeruginosa.*

The method of treating the ear infection can further comprise the administration of an antibiotic that is effective in the treatment of *P. aeruginosa* in a quantity effective to exert a bactericidal action against *P. aeruginosa,* the antibiotic being administered by a route other than route of administration of the composition according to the present invention. If the composition according to the present invention includes an antibiotic, the antibiotic that is administered by the route other than route of administration of the composition according to the present invention can be the same antibiotic included in the composition or can be a different antibiotic. If the composition according to the present invention does not include an antibiotic, the antibiotic administered by the additional route can be any of the antibiotics described above as being effective in the treatment of *P. aeruginosa.* The route of administration, dose administered, and the frequency of administration can be determined by one of ordinary skill in the art by reference to the parameters described above, such as the subject's age, size, weight, and health, the extent of the ear infection, the bacterium causing the ear infection, the presence of other conditions such as allergic reactions that can complicate the ear infection, and the therapeutics or combination of therapeutics selected for administration, as well as variables such as liver and kidney function that affect the pharmacokinetics of administered therapeutics, and the properties of the antibiotic such as its molecular weight and relative degree of hydrophobicity or hydrophilicity, as well as its susceptibility to hydrolysis in the digestive tract. Typically, administration of the antibiotic administered by the additional route is by the oral or parenteral route; if parenteral, typically the antibiotic is administered intramuscularly. In some cases of severe infection, intravenous administration can be required. Information on specific antibiotics and optimum routes of administration can be found, for example, in J.G. Hardman & L.E. Limbird, eds., "Goodman & Gilman's The Pharmacological Basis of Therapeutics" (10th ed., McGraw-Hill, New York, 2001), the relevant portions of which are incorporated herein by this reference.

### ADVANTAGES OF THE INVENTION

The present invention provides a safe and effective means for treating ear infections, particularly otitis externa and otitis media, and particularly those ear infections caused by *P. aeruginosa.* Compositions and methods according to the present invention, in removing bacteria-laden biofilm, not only provide for more effective treatment of such infections, but also prevent recurrence of the infections, such as otitis externa and otitis media. Compositions and methods according to the present invention are suitable for use with other treatment modalities, such as antibiotic administration, and do not cause inflammation or other side effects.

Accordingly, compositions and methods according to the present invention possess industrial applicability for the preparation of medicaments for the treatment of ear infections, especially otitis externa and otitis media, and especially for the treatment of infections caused by *P. aeruginosa.*

The inventions illustratively described herein can suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the future shown and described or any portion thereof, and it is recognized that various modifications are possible within the scope of the invention claimed. Thus, it should be understood that although the present invention has been specifically disclosed by preferred embodiments and optional features, modification and variation of the inventions herein disclosed can be resorted by those skilled in the art, and that such modifications and variations are considered to be within the scope of the inventions disclosed herein. The inventions have been described broadly and generically herein. Each of the narrower species and subgeneric groupings falling within the scope of the generic disclosure also form part of these inventions. This includes the generic description of each invention with a proviso or negative limitation removing any subject matter from the genus, regardless of whether or not the excised materials specifically resided therein.

In addition, where features or aspects of an invention are described in terms of the Markush group, those schooled in the art will recognize that the invention is also thereby described in terms of any individual member or subgroup of members of the Markush group. It is also to be understood that the above description is intended to be illustrative and not restrictive. Many embodiments will be apparent to those of ordinary skill in the art upon reviewing the above description. The scope of the invention should therefore, be determined not with reference to the above description, but should instead be determined with reference to the appended claims, along with the full scope of equivalents to which such claims are entitled. The disclosures of all articles and references, including patent publications, are incorporated herein by reference.

## Claims

1. Use of a composition comprising a quantity of at least one enzyme that catalyzes the hydrolysis of a bond that connects two monosaccharides in a polysaccharide or that connects a monosaccharide with a protein molecule in a glycoprotein sufficient to break down biofilm in the ear for treating an ear infection comprising the step of administering a quantity of the composition to a subject with an ear infection in order to treat the ear infection.

2. The use of claim 1 wherein the at least one enzyme that catalyzes the hydrolysis of a bond that connects two monosaccharides in a polysaccharide or that connects a monosaccharide with a protein molecule in a glycoprotein is selected from the group consisting of dextranase, pectinase, and β-D-glucosidase.

3. The use of claim 1 wherein the at least one enzyme that catalyzes the hydrolysis of a bond that connects two monosaccharides in a polysaccharide or that connects a monosaccharide with a protein molecule in a glycoprotein is selected from the group consisting of dextranase, pectinase, and cellulase.

4. The use of claim 1 wherein the composition further includes an aminohexose, wherein the aminohexose is aminoglucose.

5. The use of claim 4 wherein the aminoglucose is selected from the group consisting of glucosamine, N-acetylglucosamine, and mixtures thereof.

6. The use of claim 1 wherein the composition further includes an oxidase in a bactericidally effective quantity.

7. The use of claim 6 wherein the composition further includes a substrate for the oxidase in a bactericidally effective quantity.

8. The use of claim 6 wherein the oxidase is glucose oxidase.

9. The use of claim 8 wherein the composition further includes glucose as a substrate for the glucose oxidase in a bactericidally effective quantity.

10. The use of claim 1 wherein the ear infection is otitis media

11. The use of claim 1 wherein the ear infection is otitis externa.

12. The use of claim 1 wherein the ear infection is caused by *Pseudomonas aeruginosa.*

13. The use of claim 12 wherein the use further comprises administering an antibiotic that is effective in the treatment of *P. aeruginosa* in a quantity effective to exert a bactericidal action against *P. aeruginosa,* the antibiotic being administered by a route other than the route of administration of the composition.

14. The use of claim 12 wherein the composition further comprises an antibiotic that is effective in the treatment of *P. aeruginosa* in a quantity effective to exert a bactericidal action against *P. aeruginosa.*

15. The method of claim 14 wherein the antibiotic that is administered by the route other than the route of administration of the composition is the same as the antibiotic included in the composition.
